(19) Europäisches Patentamt
European Patent Office
Office européen des brevets

(11) EP 4 692 096 A1

(12) **EUROPEAN PATENT APPLICATION**
published in accordance with Art. 153(4) EPC

(43) Date of publication:
**11.02.2026 Bulletin 2026/07**

(21) Application number: **24778264.2**

(22) Date of filing: **29.03.2024**

(51) International Patent Classification (IPC):
*C07D 498/22 (2006.01)* *C07D 498/18 (2006.01)*
*C07D 487/22 (2006.01)* *C07D 487/18 (2006.01)*
*A61K 31/5025 (2006.01)* *A61P 37/02 (2006.01)*
*A61P 29/00 (2006.01)*

(52) Cooperative Patent Classification (CPC):
**A61K 31/5025; A61P 29/00; A61P 37/02; C07D 487/18; C07D 487/22; C07D 498/18; C07D 498/22**

(86) International application number:
**PCT/CN2024/084966**

(87) International publication number:
**WO 2024/199479 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.03.2023 CN 202310338361**

(71) Applicant: **Prime Gene Therapeutics Co., Ltd.**
**Beijing 100070 (CN)**

(72) Inventors:
- **ZHU, Li**
  **Beijing 100070 (CN)**
- **HU, Wei**
  **Beijing 100070 (CN)**
- **DAI, Liguang**
  **Beijing 100070 (CN)**
- **DU, Daniel Yunlong**
  **Beijing 100070 (CN)**

(74) Representative: **Winter, Brandl - Partnerschaft mbB**
**Alois-Steinecker-Straße 22**
**85354 Freising (DE)**

# (54) MACROCYCLIC COMPOUND, AND PHARMACEUTICAL COMPOSITION AND USE THEREOF

(57) The present disclosure relates to a macrocyclic compound, a pharmaceutical composition and use thereof. The compound has a structural formula as shown in Formula I. The macrocyclic compound is a highly selective TYK2 allosteric inhibitor with strong blood-brain barrier penetration that can be used to regulate TYK2-mediated diseases.

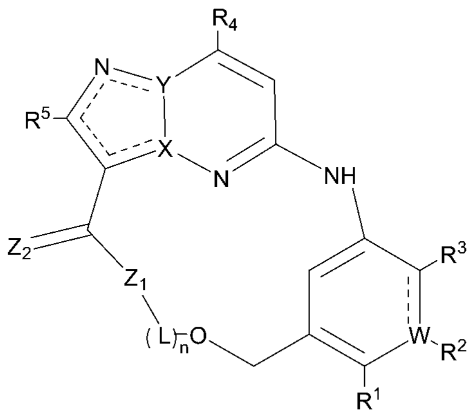

I

EP 4 692 096 A1

## Description

### Technical Field

The present application provides a macrocyclic compound, a pharmaceutical composition comprising the compound, and uses thereof. The macrocyclic compound is a highly selective TYK2 allosteric inhibitor with strong blood-brain barrier penetration that can be used to regulate TYK2-mediated diseases.

### Background

The Janus kinase (JAK) family plays a critical role in mediating the signal transduction of numerous cytokines that cause inflammation, and includes JAK1, JAK2, JAK3, and TYK2. TYK2 and JAK1/2/3 typically function in pairs, also referred to as "dimers," transmitting extracellular cytokine signals into the cell nucleus. TYK2 selectively participates in signaling pathways triggered by pro-inflammatory cytokines such as IL-23, IL-12 and type I interferons (IFNs). Accordingly, TYK2 inhibitors have emerged as promising therapeutic agents for a wide range of severe inflammatory and autoimmune diseases.

JAK1, JAK2, JAK3 and TYK2 of the JAK family possess JAK homology domains (JH), in which the JH1 domain is also also known as the kinase domain and the JH2 domain is known as the pseudokinase domain. Early TYK2 inhibitors, such as Pfizer's Brepocitinib, target the JH1 kinase catalytic domain, i.e. ATP-binding pocket. Due to the high degree of homology across JH1 catalytic domains of JAK family members, these inhibitors exhibit limited selectivity for other members of the JAK family, making it difficult to avoid common JAK inhibitors related adverse events, such as cardiovascular incidents and venous thromboembolism.

Bristol Myers Squibb (BMS)'s TYK2 allosteric inhibitor Deucravacitinib binds to a specific pocket within the TYK2 JH2 pseudokinase domain, and achieves high selectivity over other JAK family members and the whole kinase family by way of allosteric inhibition, accordingly reducing the risk of adverse events, such as cardiovascular incidents and venous thromboembolism, so that Deucravacitinib has become the first oral JAK inhibitor to be approved without a boxed warning.

However, Deucravacitinib still exhibits certain activity against the JH2 domain of JAK1, and the inhibitory for JAK1 poses a risk of JAK1-related adverse effects. The development of a TYK2 allosteric inhibitor with higher selectivity against JAK1 could further reduce such risks, offering safer therapeutic options for autoimmune diseases. Moreover, many autoimmune diseases, in their advanced stages, may invade the brain, leading to symptoms such as multifocal or diffuse brain damage. Currently, there are no highly selective TYK2 inhibitors reported with strong brain penetration, leaving a significant unmet clinical need. In addition, TYK2 is an activator of the STAT pathway in response to multiple pro-inflammatory cytokines. Inhibiting TYK2 may help reduce inflammation in neurodegenerative diseases such as Parkinson's disease, Alzheimer's disease, amyotrophic lateral sclerosis (ALS), and multiple sclerosis (MS). Thus, there is an urgent clinical need for the development of a highly selective TYK2 allosteric inhibitor with strong blood-brain barrier penetration as a therapeutic agent for neurodegenerative diseases.

### Summary of the Invention

In one aspect, the present disclosure provides a compound shown in Formula I, isotope isomers or pharmaceutically acceptable salts thereof:

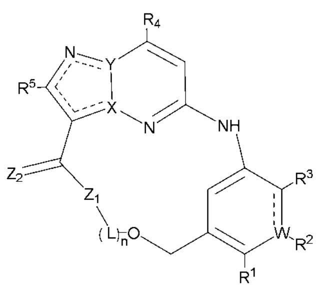

Formula I.

The compound shown in Formula I is a highly selective TYK2 allosteric inhibitor with strong blood-brain barrier penetration, suitable for the modulation of TYK2-mediated diseases.

The present disclosure also relates to a pharmaceutical composition, comprising the compound shown in Formula I, or pharmaceutically acceptable salts, hydrates, solvates, active metabolites, polymorphs, isotopic markers,

isomers or prodrugs thereof, and a pharmaceutically acceptable carrier.

Further, the present disclosure relates to use of the compound shown in Formula I or pharmaceutically acceptable salts, hydrates, solvates, active metabolites, polymorphs, isotopic markers, isomers or prodrugs thereof, as well as the pharmaceutical composition, in the preparation of pharmaceuticals for treating tyrosine kinase-mediated diseases.

The present disclosure also provides a method for treating tyrosine kinase mediated diseases, comprising administering a therapeutically effective amount of the compound shown in Formula I or pharmaceutically acceptable salts, hydrates, solvates, active metabolites, polymorphs, isotopic markers, isomers or prodrugs thereof, or the pharmaceutical composition, to a subject in need.

In one embodiment, the tyrosine kinase is selected from TYK2 kinases. In one embodiment, the tyrosine kinase mediated diseases include inflammatory autoimmune diseases, such as atopic dermatitis, hidradenitis suppurativa, psoriasis, psoriatic arthritis, Crohn's disease, ulcerative colitis, lupus erythematosus, scleroderma, and autoimmune encephalopathy; tumors, such as leukemia, lymphoma, myeloma, and brain tumor; and neurodegenerative diseases, such as brain atrophy, dementia, Parkinson's syndrome, Alzheimer's disease, amyotrophic lateral sclerosis, and multiple sclerosis.

## Detailed Description

The present application will be further explained below in detail through embodiments. Through these descriptions, the features and advantages of the present application will become more clearly defined.

The term "exemplary" used here means "serving as an example, embodiment, or illustration." Any embodiment described here as "exemplary" should not be interpreted as being superior or better than other embodiments.

Furthermore, the technical features in different embodiments of the present application described below can be combined with each other if they do not conflict.

### Definitions

Unless defined otherwise, all technical and scientific terms used herein have the same meaning as that commonly understood by one of skill in the art to which the claimed subject matter belongs. All patents, patent applications, published materials referred to throughout the entire disclosure herein, unless noted otherwise, are incorporated by reference in their entirety. When a trade name appears herein, it is intended to refer to its corresponding product or its active ingredient.

It is to be understood that the foregoing general description and the following detailed description are exemplary and explanatory only, and are not restrictive of any subject matter claimed. In the present application, it must be noted that unless otherwise explicitly stated in the text, the singular used in the present description and claims includes the plural of the referred object. It should also be noted that "or" used herein means "and/or" unless stated otherwise. Furthermore, the term "comprise(s)" used herein as well as other forms, such as "comprising", "contain(s)", and "include(s)" is not limiting.

Definitions of standard chemical terms may be found in the literature, including Carey and Sundberg's "Advanced Organic Chemistry 4th Ed, Vol A (2000) and B (2001), Plenum Press, New York. Unless otherwise specified, conventional methods within the technical field are applied, such as mass spectrometry, NMR, HPLC, protein chemistry, biochemistry, recombinant DNA technology, and pharmacological methods. Unless specific definitions are provided, those skilled in the art know the related terms and laboratory operations and techniques in chemistry such as analytical chemistry, synthetic organic chemistry, and medical and pharmaceutical chemistry used herein. Standard techniques may be used for chemical synthesis, chemical analysis, drug preparation, formulation, drug delivery and patient treatment. Standard techniques may be used for recombinant DNA, oligonucleotide synthesis, and tissue culture and transformation (such as electroporation, lipid infection). For example, a kit with instructions provided by the manufacturer may be used, or the reaction and purification techniques may be carried out according to methods known in the art, or according to the method described in the present disclosure. Generally speaking, the aforementioned techniques and steps may be implemented by conventional methods well-known in the art and described in various general documents or more specific documents. These documents are described and cited in the present disclosure.

When a substituent is described by a conventional chemical formula written from left to right, the substituent also includes chemically equivalent substituents obtained when the structural formula is written from right to left. For example, $CH_2O$ is equivalent to $OCH_2$.

The term "substituted" means that any one or more hydrogen atoms on a specific atom are replaced by a substituent, as long as the valence of the specific atom is normal and the compound after substitution is stable. When the substituent is oxo (i.e. =O), it means that two hydrogen atoms are replaced, which will not occur on aromatic groups.

When any variable (such as R) occurs more than once in the composition or structure of a compound, its definition in each case is independent. Thus, for example, if a group is substituted with 0-2 R, the group may optionally be substituted

with up to two R, and R has independent options in each case. In addition, combinations of substituents and/or variants thereof are only permitted if such combinations result in stable compounds.

As used herein, $C_{m-n}$ refers to the part having m-n carbon atoms. For example, the "$C_{1-8}$" group means that the part has 1-8 carbon atoms, that is, the group contains 1 carbon atom, 2 carbon atoms, 3 carbon atoms... 8 carbon atoms. Therefore, for example, "$C_{1-8}$ alkyl" refers to an alkyl containing 1-8 carbon atoms, that is, the alkyl is selected from the group consisting of methyl, ethyl, propyl, isopropyl, n-butyl, isobutyl, sec-butyl, tert-butyl... octyl, etc. Numerical ranges herein, for example "1-8" refers to each integer within the given range. For example, "1-8 carbon atoms" means that the group may have 1 carbon atom, 2 carbon atoms, 3 carbon atoms, 4 carbon atoms, 5 carbon atoms, 6 carbon atoms, 7 carbon atoms, or 8 carbon atoms.

The term "membered" refers to the number of skeletal atoms forming a ring. For example, pyridine is a six-membered ring, and pyrrole is a five-membered ring.

In this disclosure, various groups may have the following definitions:

Hydrogen may be represented by -H or replaced by isotopes such as deuterium and tritium.

Halogen may include fluorine, chlorine, bromine, and iodine.

$C_{1-8}$ alkyl groups may include methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, n-butyl, isobutyl, sec-butyl, tert-butyl, n-pentyl, isopentyl, neopentyl, tert-pentyl, hexyl, heptyl, octyl, etc.

Deuterated or tritiated $C_{1-8}$ alkyl groups may represent $C_{1-8}$ alkyl groups in which one or more, or even all, hydrogen atoms are replaced by deuterium, tritium, or other isotopes.

$C_{1-8}$ alkoxy groups may be represented by $-OC_{1-8}$ alkyl, where the $C_{1-8}$ alkyl group includes the groups defined above. For example, $C_{1-8}$ alkoxy groups may include methoxy, ethoxy, n-propoxy, isopropoxy, n-butoxy, isobutoxy, sec-butoxy, and tert-butoxy.

$C_{1-8}$ haloalkyl groups may represent $C_{1-8}$ alkyl groups in which any number of hydrogen atoms are replaced by halogen, where the $C_{1-8}$ alkyl group and halogen group include the groups defined above. For example, $C_{1-8}$ haloalkyl groups may include $-CF_3$.

$C_{3-8}$ cycloalkyl groups may represent non-aromatic saturated carbocyclic rings, including monocyclic (one ring) and bicyclic (two ring) carbocyclic rings. For example, $C_{3-8}$ cycloalkyl groups may include

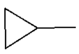, 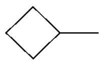, 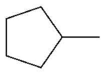, 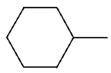, and 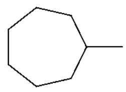.

$C_{3-8}$ cycloalkyl $C_{1-8}$ alkyl groups may represent $C_{1-8}$ alkyl groups with a $C_{3-8}$ cycloalkyl group, where the definitions of $C_{3-8}$ cycloalkyl and $C_{1-8}$ alkyl are as described above. For example, $C_{3-8}$ cycloalkyl $C_{1-8}$ alkyl groups may include cyclopropylmethyl, cyclobutylmethyl, and cyclohexylethyl, and the like.

$C_{3-8}$ heterocyclic groups may represent $C_{3-8}$ cycloalkyl groups in which any number of ring atoms are substituted with heteroatoms such as O, S, N, P, or Si, where the $C_{3-8}$ cycloalkyl group includes groups as defined above. For example, $C_{3-8}$ heterocyclic groups may include oxiranyl, thioranyl, aziridinyl, azetidinyl, oxetanyl, thietanyl, tetrahydrofuranyl, pyrrolidinyl, oxazolidinyl, tetrahydropyrazolyl, pyrrolinyl, dihydrofuranyl, dihydrothiophenyl, piperidinyl, tetrahydropyranyl, tetrahydrothiopyranyl, morpholinyl, piperazinyl, dihydropyridinyl, tetrahydropyridinyl, dihydropyranyl, tetrahydropyranyl, dihydrothiopyranyl, azepanyl, oxetanyl, thiepanyl, oxazabicyclo[2.2.1]heptyl, azaspiro[3.3]heptyl, and the like.

$C_{6-20}$ aryl groups may include monocyclic aryl groups, bicyclic aryl groups, or multicyclic aryl groups, for example, phenyl, biphenyl, naphthyl, phenanthrenyl, anthracenyl, azulenyl, and the like.

$C_{5-20}$ heteroaryl groups may represent unsaturated groups containing any number of heteroatoms such as O, S, N, P, or Si as ring atoms. For example, $C_{5-20}$ heteroaryl groups may include pyrrolyl, furanyl, thienyl, imidazolyl, oxazolyl, pyrazolyl, pyridyl, pyrimidinyl, pyrazinyl, quinolyl, isoquinolyl, tetrazolyl, triazolyl, triazinyl, benzofuranyl, benzothienyl, indolyl, isoindolyl, and the like.

Hydroxyl group may be represented by -OH.

Thiol group may be represented by -SH.

Carboxyl group may be represented by -COOH.

Ester group may be represented by -COOR', wherein R' can be a $C_{1-8}$ alkyl group. For example, an ester group substituted with a $C_{1-8}$ alkyl group may be represented by $-COOC_{1-8}$ alkyl, where the $C_{1-8}$ alkyl group includes the groups defined above.

Acyl group may be represented by -COR', wherein R' can be a $C_{1-8}$ alkyl group. For example, an acyl group substituted with a $C_{1-8}$ alkyl group may be represented by $-COC_{1-8}$ alkyl, where the $C_{1-8}$ alkyl group includes the groups as defined above.

Amino group may be represented by $-NH_2$, -NHR', or $-N(R')_2$, wherein R' can be a $C_{1-8}$ alkyl group. For example,

an amino group substituted with a $C_{1-8}$ alkyl group can be represented by - $NHC_{1-8}$ alkyl or $-N(C_{1-8}$ $alkyl)_2$, where the $C_{1-8}$ alkyl group includes the groups as defined above.

Amide group may be represented by -CO amino, where the amino group includes the groups defined above.

Sulfonyl group may be represented by $-S(O)_2R'$, wherein R' can be a $C_{1-8}$ alkyl group. For example, a sulfonyl group substituted with a $C_{1-8}$ alkyl group can be represented by $-S(O)_2C_{1-8}$ alkyl, where the $C_{1-8}$ alkyl group includes the groups as defined above.

Cyano group may be represented by -CN.

Oxo group may be represented by =O.

In the foregoing definitions, when the number of carbon atoms varies, the change applies only to the carbon count and does not alter the type of group defined. For example, $C_{1-5}$ alkyl groups may include methyl, ethyl, n-propyl, isopropyl, n-butyl, isobutyl, sec-butyl, tert-butyl, n-pentyl, isopentyl, neopentyl, and all other groups within the range of "$C_{1-8}$ alkyl" definition that contain 1-5 carbons.

The term "pharmaceutically acceptable" refers to those compounds, materials, compositions and/or dosage forms that are within the scope of reliable medical judgment and are suitable for use in contact with human and animal tissues without excessive toxicity, irritation, allergic reactions or other problems or complications of the disease, and are commensurate with a reasonable benefit/risk ratio.

The term "pharmaceutically acceptable salt" refers to a salt that retains the biological efficacy of the free acid and free base of the specified compound and has no adverse effects in biology or other aspects. Unless otherwise specified, the salts in the present disclosure may comprise metal salts, ammonium salts, salts formed with organic bases, salts formed with inorganic acids, salts formed with organic acids, salts formed with basic or acidic amino acids, etc. Non-limiting examples of metal salts comprise, but are not limited to, alkali metal salts, such as sodium salt, potassium salt, etc.; alkaline earth metal salts, such as calcium salt, magnesium salt, barium salt, etc.; aluminum salt, and the like. Non-limiting examples of salts formed with organic bases comprise, but are not limited to, the salts formed with trimethylamine, triethylamine, pyridine, picoline, 2,6-lutidine, ethanolamine, diethanolamine, triethanolamine, cyclohexylamine, dicyclohexylamine and the like. Non-limiting examples of salts formed with inorganic acids comprise, but are not limited to, salts formed with hydrochloric acid, hydrobromic acid, nitric acid, sulfuric acid, phosphoric acid, and the like. Non-limiting examples of salts formed with organic acids comprise, but are not limited to, salts formed with formic acid, acetic acid, trifluoroacetic acid, fumaric acid, oxalic acid, malic acid, maleic acid, tartaric acid, citric acid, succinic acid, methanesulfonic acid, benzene sulfonic acid, p-toluenesulfonic acid, etc. Non-limiting examples of salts formed with basic amino acids comprise, but are not limited to, salts formed with arginine, lysine, ornithine, and the like. Non-limiting examples of salts formed with acidic amino acids comprise, but are not limited to, salts formed with aspartic acid, glutamic acid, and the like.

Pharmaceutically acceptable salts may be synthesized from parent compounds containing acid radicals or bases by conventional chemical methods. Generally, such salts are prepared by reacting these compounds in free acid or base form with a stoichiometric amount of appropriate base or acid in water or organic solvent or a mixture of both. Generally, non-aqueous media such as ether, ethyl acetate, ethanol, isopropanol or acetonitrile are preferred.

The term "solvate" refers to a physical aggregate formed by a compound of the present imvention and one or more solvent molecules. This physical aggregate comprises varying degrees of ions and covalent bonds, such as hydrogen bonds. It has been shown that this solvate may be separated, for example, when one or more solvent molecules are mixed in the crystal lattice. "solvate" comprises both solvent phase and separable solvate. There are many examples of corresponding solvates, including ethanol solvates, methanol solvates and the like. "Hydrate" is a solvate that uses water ($H_2O$) molecules as a solvent. One or more compounds in the present disclosure may be prepared as solvates at will. The preparation of solvates is well known. For example, M. Caira et al, J. Pharmaceutical Sci., 93(3), 601-611 (2004) describe the preparation of a solvate of the antifungal drug fluconazole, that is, preparation with ethyl acetate and water. E.C. van Tonder et al, AAPS PharmSciTech., 5(1), article 12 (2004); and AL Bingham et al, Chem. Commun., 603-604 (2001) also describes the similar methods for preparing solvates and hydrates. A typical, non-limiting preparation process is to dissolve the compound of the present disclosure in a required amount of ideal solvent (organic solvent or water or their mixed solvent) at a temperature higher than normal temperature, to cool down, and to leave to crystallize. Then, the crystals are separated by using standard methods. I. R. spectroscopy analysis technique may confirm the existence of the solvent (water) that forms the solvate (hydrate) in the crystal.

The term "active metabolite" refers to an active derivative of the compound formed when the compound is metabolized.

The term "polymorphs" refers to compounds of the present disclosure that exist in different crystal lattice forms.

The term "isotopic marker" refers to an isotopically marked compound of the present disclosure. For example, the isotopes in the compound of the present disclosure may comprise various isotopes of elements such as H, C, N, O, P, F, S, such as $^{2}H$, $^{3}H$, $^{13}C$, $^{14}C$, $^{15}N$, $^{18}O$, $^{17}O$, $^{31}P$, $^{32}P$, $^{35}S$, $^{18}F$ and $^{36}S$.

The term "pharmaceutically acceptable prodrug" or "prodrug" refers to any pharmaceutically acceptable salt, ester, ester salt or other derivative of the compound of the present disclosure, which may directly or indirectly provide the

compound of the present disclosure or its pharmaceutically active metabolite or residue after administration to a recipient. Particularly preferred derivatives or prodrugs are those compounds that may improve the bioavailability of the compounds of the present disclosure when administered to patients (for example, may make oral compounds more easily absorbed into the blood), or promote the parent compound delivering to biological organs or the site of action, such as the brain or lymphatic system. The functional groups in the compound may be modified by conventional operations or in vivo in a modification manner that may be decomposed into the parent compound to prepare a prodrug. Various prodrug forms are well known in the art. See, Pro-drugs as Novel Delivery Systems (1987) Vol. 14 of the ACS Symposium Series by T. Higuchi and V. Stella, Bioreversible Carriers in Drug Design, (1987) Edward B. Roche, ed., American Pharmaceutical Association and Pergamon Press provide discussions on prodrugs. Design of Prodrugs, Bundgaard, A. Ed., Elseview, 1985 and Method in Enzymology, Widder, K. et al., Ed.; Academic, 1985, vol. 42, p. 309-396 ; Bundgaard, H. "Design and Application of Prodrugs" in A Textbook of Drug Design and Development, Krosgaard-Larsen and H. Bundgaard, Ed., 1991, Chapter 5, pp. 113-191 ; and Bundgaard, H., Advanced Drug Delivery Review, 1992, 8, 1-38 , the above documents are incorporated herein by reference.

The term "stereoisomers" refers to isomers produced by different arrangements of atoms in the molecule in space. The compounds of the present disclosure contain structures such as asymmetric or chiral centers and double bonds. Therefore, the compounds of the present disclosure may comprise optical isomers, geometric isomers, tautomers, atropisomers and other isomers. These isomers and their single isomers, racemates, etc. are all included in the scope of the present disclosure. For example, for optical isomers, optically active (R)- and (S)-isomers and D and L isomers may be prepared by chiral resolution, chiral synthesis or chiral reagents or other conventional techniques. For example, it may be converted into diastereomers by reacting with appropriate optically active substances (such as chiral alcohols or Mosher's chloride), separated and converted (such as hydrolyzed) into the corresponding single isomer. As another example, it may also be separated by a chromatographic column.

The term "pharmaceutical composition" refers to a biologically active compound optionally mixed with at least one pharmaceutically acceptable chemical component or agent. The pharmaceutically acceptable chemical component or agent is the "carrier", which helps for introducing the compound into cells or tissues. It comprises, but is not limited to, stabilizers, diluents, suspending agents, thickeners, and/or excipients.

The "pharmaceutical compositions" herein may be prepared in a manner well known in the pharmaceutical field, and they may be administered or applied by various routes, depending on whether local or systemic treatment is required and the area to be treated. It may be topically administered (for example, transdermal, skin, eye and mucous membranes including intranasal, vaginal and rectal delivery), pulmonarily administered (for example, by inhalation or insufflation of powder or aerosol, including through sprayers; intratracheal, intranasal), orally or parenterally administered. Parenteral administration includes intravenous, intraarterial, subcutaneous, intraperitoneal or intramuscular injection or infusion; or intracranial, such as intrathecal or intracerebroventricular administration. It may be administered parenterally in a single bolus dose, or it may be administered by, for example, a continuous infusion pump. The pharmaceutical composition herein comprises but is not limited to the following forms: tablets, pills, powders, lozenges, sachets, cachets, elixirs, suspensions, emulsions, solutions, syrups, aerosols (solid or dissolved in a liquid vehicle); for example, ointments, soft and hard gelatin capsules, suppositories, sterile injection solutions and sterile packaged powders containing up to 10% by weight of the active compound.

The pharmaceutical composition herein may be formulated in a unit dosage form, and each dosage may contain about 0.1 to 1000 mg, usually about 5 to 1000 mg of active ingredient, and more usually about 100 to 500 mg of active ingredient. The term "unit dosage form" refers to a physically separated single dosage unit suitable for use in human patients and other mammals, each unit containing a predetermined amount of active substance mixed with a suitable pharmaceutical carrier calculated to produce the desired therapeutic effect.

The term "individual" refers to an individual suffering from a disease, disorder, or condition, and comprises mammals and non-mammals. Examples of mammals comprise, but are not limited to, any member of the class Mammals: humans, non-human primates (such as chimpanzees and other apes and monkeys); domestic animals, such as cows, horses, sheep, goats, and pigs; domesticated animals, such as rabbits, dogs, and cats; laboratory animals, comprising rodents, such as rats, mice, and guinea pigs.

The term "treatment" and other similar synonyms comprise alleviation, reduction or amelioration of symptoms of a disease or condition, prevention of other symptoms, amelioration or prevention of the underlying metabolic cause of the symptoms, inhibition of the disease or condition, such as preventing the development of the disease or condition, alleviating the disease or condition, making a disease or condition better, alleviating the symptoms caused by the disease or condition, or stoping the symptoms of the disease or condition. In addition, the term may also comprise the purpose of prevention. The term also comprises obtaining therapeutic effects and/or preventive effects. The therapeutic effect refers to curing or improving the underlying disease being treated. In addition, the cure or improvement of one or more physiological symptoms associated with the underlying disease is also a therapeutic effect. For example, although the patient may still be affected by the underlying disease, an improvement in the patient's condition is observed. In terms of preventive effects, the composition or compound may be administered to patients who are at risk of suffering from a

specific disease, or even if a disease diagnosis has not been made, the composition or compound may be administered to patients who have one or more physiological symptoms of the disease.

The term "amount to obtain the necessary therapeutic effect" or "therapeutically effective amount" refers to the amount of at least one agent or compound sufficient to relieve one or more symptoms of the disease or condition being treated after administration. The result may be a reduction and/or alleviation of signs, symptoms or causes, or any other desired changes in the biological system. Techniques such as dose escalation tests may be used to determine the effective amount suitable for any individual case. The actual amount of compound, pharmaceutical composition or agent to be administered is usually determined by the physician according to the relevant circumstances, comprising the condition being treated, the route of administration selected, the actual compound administered; the age, weight and response of the individual patient; and the severity of the patient's symptoms, etc.

The ratio or concentration of the compound of the present disclosure in the pharmaceutical composition may not be fixed, depending on various factors, comprising dosage, chemical properties (for example, hydrophobicity), route of administration, and the like. For example, the compound of the present disclosure may be provided by a physiologically buffered aqueous solution containing about 0.1-10% w/v of the compound for parenteral administration. Some typical dosage ranges are from about 1 μg/kg to about 1 g/kg body weight/day. In certain embodiments, the dosage range is from about 0.01 mg/kg to about 100 mg/kg body weight/day. The dosage is likely to depend on such variables, such as the type and degree of development of the disease or condition, the general health status of the specific patient, the relative biological efficacy of the selected compound, the excipient formulation and its route of administration.

The term "administration" refers to a method capable of delivering a compound or composition to a desired site for biological action. These methods comprise, but are not limited to, oral routes, transduodenal routes, parenteral injections (comprising intravenous, subcutaneous, intraperitoneal, intramuscular, intraarterial injection or infusion), topical, and rectal administration. Those skilled in the art are familiar with administration techniques that may be used for the compounds and methods described herein, for example, those discussed in Goodman and Gilman, The Pharmacological Basis of Therapeutics, currented.; Pergamon; and Remington's, Pharmaceutical Sciences (current edition), Mack Publishing Co., Easton, Pa.

The term "$IC_{50}$" refers to 50% inhibition of the maximum effect obtained in an analysis measuring such an effect.

To make the purposes, technical configurations and advantages of the present disclosure clearer, the technical configurations of exemplary embodiments of the present disclosure are further described below.

Compounds

The present disclosure provides a compound shown in Formula I, or pharmaceutically acceptable salts, hydrates, solvates, active metabolites, polymorphs, isotopic markers, isomers or prodrugs thereof:

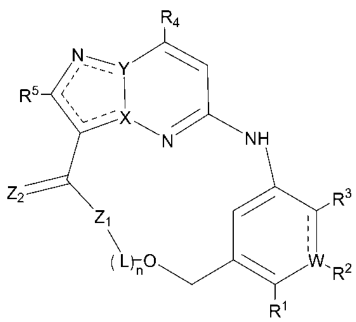

Formula I

wherein X and Y are each independently selected from the group consisting of N and C atoms, and one is N and the other is C; W is selected from the group consisting of N and C atoms; $Z_1$ is independently selected from the group consisting of O and S atoms; $Z_2$ is selected from the group consisting of O, S and NR, wherein R is H or $C_{1-6}$ alkyl; ------ represents single or double bond; $R^1$, $R^2$ and $R^3$ are each independently selected from the group consisting of hydrogen, halogen, oxo, $C_{1-6}$ alkyl, and $C_{1-6}$ alkoxy, or $R^2$ and $R^3$, together with the ring atoms to which they are attached, form a 5- or 6-membered heteroaryl ring substituted with 0-2 substituents $R^{23}$; $R^4$ is selected from the group consisting of $NR^{41}R^{42}$, $C_{1-6}$ alkyl, and $C_{1-6}$ alkoxy; $R^5$ is selected from the group consisting of hydrogen and $NR^{51}R^{52}$; L is each independently selected from $-CR^{L1}R^{L2}-$. $R^{L1}$ and $R^{L2}$ on each L are each independently selected from the group consisting of hydrogen and $C_{1-6}$ alkyl, or two $R^{L2}$ on the two adjacent L's, together with the carbons of L to which they are attached, form a $C_{3-6}$ cycloalkyl ring substituted with 0-2 $R^{23}$; $R^{23}$ is each independently selected from the group consisting of halogen, $C_{1-6}$ alkyl, and $C_{1-6}$ alkoxy; $R^{41}$ and $R^{42}$ are each independently selected from hydrogen and $C_{1-6}$ alkyl; $R^{51}$ and $R^{52}$ are each independently

selected from hydrogen and $C_{1-6}$ alkyl; and n is an integer from 2 to 5.

In the five-membered ring containing X and Y, ------ can be a single or double bond according to the selection of X and Y, such that the five-membered ring has a heteroaryl ring structure. Depending on the selection of X and Y, the compound may have a structure formula of Formula IIa or Formula IIb:

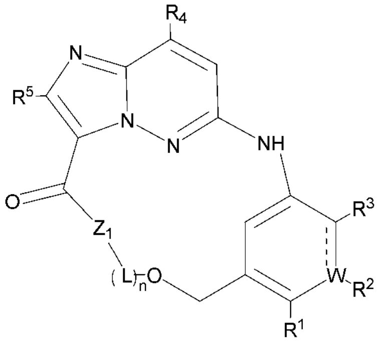

Formula IIa

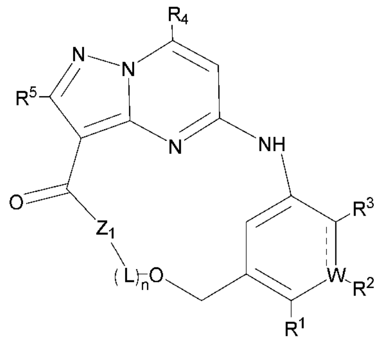

Formula IIb

wherein $R^1$, $R^2$, $R^3$, $R^4$, $R^5$, W, L, and n are as defined above.

In Formulas I, IIa, and IIb, when W is a carbon atom, the bond between W and the carbon atom attached to $R^3$ is a double bond, i.e., the six-membered ring containing W is a benzene ring.

In one embodiment, $Z_1$ is O or S atom.

In one embodiment, $R^4$ is $NR^{41}R^{42}$, wherein $R^{41}$ is hydrogen and $R^{42}$ is $C_{1-3}$ alkyl group. In one embodiment, $R^4$ is $NR^{41}R^{42}$, wherein $R^{41}$ is hydrogen and $R^{42}$ is methyl group, i.e., $R^4$ is methylamino.

In one embodiment, $R^5$ is hydrogen or $-NH_2$.

In one embodiment, n is 2 or 3, and L is each independently selected from $-CR^{L1}R^{L2}-$, wherein $R^{L1}$ on each L is hydrogen, and $R^{L2}$ on each L is each independently selected from the group consisting of hydrogen and methyl. Preferably, among the (L)n formed by n L's, exactly one $R^{L2}$ is methyl.

In one embodiment, n is 2 or 3, and L is each independently selected from $-CR^{L1}R^{L2}-$, wherein $R^{L1}$ on each L is hydrogen, two $R^{L2}$ on two adjacent L together with carbon atoms of L attached thereto form a $C_{3-6}$ cycloalkyl ring, such as cyclobutyl or cyclopentyl.

In one embodiment, (L)n formed by n L's is selected from $*-CH_2CH_2-**$, $*-CH_2CH_2CH_2-**$, $*-CH(CH_3)CH_2-**$, $*-CH_2CH(CH_3)-**$, $*-CH(CH_3)CH_2CH_2-**$,

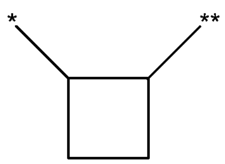

or

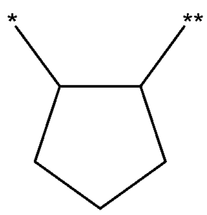

,

wherein * represents the site connected to atom $Z_1$, and ** represents the site connected to oxygen atom.

In one embodiment, W is N, and preferably, in the embodiment where W is N, $R^1$ is H, $R^2$ is H or $C_{1-3}$ alkyl, and $R^3$ is oxo.

In one embodiment, W is C, and preferably, in the embodiment where W is C, $R^1$ is H or halogen, $R^2$ is H or halogen, and $R^3$ is $C_{1-3}$ alkoxy. In one embodiment, W is C, $R^1$ is H, $R^2$ is F, and $R^3$ is methoxy. In one embodiment, W is C, $R^1$ is F, $R^2$ is H, and $R^3$ is methoxy. In one embodiment, W is C, $R^1$ is H, $R^2$ is H, and $R^3$ is methoxy. In these embodiments, thebetween W and the carbon atom attached to $R^3$ is a double bond, i.e., the six-membered ring containing W is a benzene

ring.

In one embodiment, W is C, $R^2$ and $R^3$ together with the ring atoms attached thereto form a five- or six-membered heteroaryl ring (e.g., triazole) substituted with 0-2 $R^{23}$.

In one embodiment, the moiety

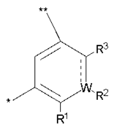

of above Formula I, IIa and IIb is

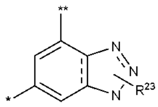,

wherein $R^{23}$ is selected from the group consisting of hydrogen and $C_{1-3}$ alkyl, * represents the site connected to $CH_2$, ** represents the site connected to NH.

Thus, the compound of the present disclosure may have the structure as shown by any one of the following general formulas:

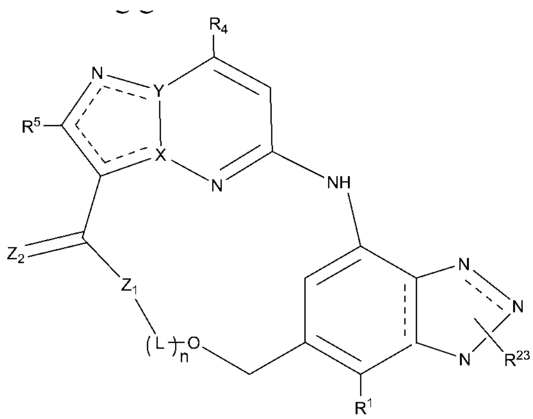

Formula I-1,

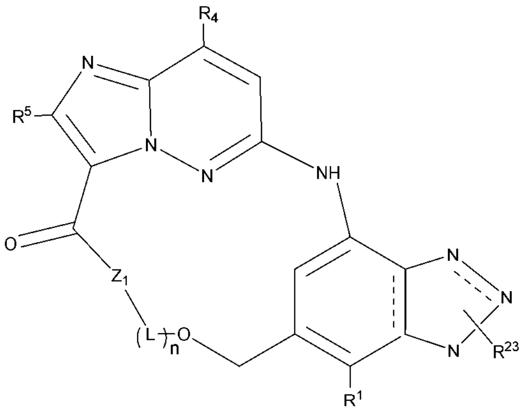

Formula IIa-1,

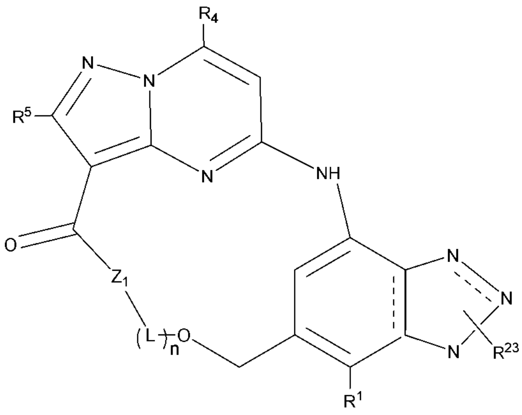

Formula IIb-1;

wherein the groups are as defined above.

In the above Formula I-1, $Z_2$ is preferably O.

It should be noted that, in the above Formulas I-1, IIa-1 and IIb-1, the position of the double bond in the benzotriazole structure will change with the different substitution positions of $R^{23}$ on the triazole ring, as long as the double bond in the benzotriazole ring structure is in the form of a conjugated double bond.

For example, depending on the different substitution positions of $R^{23}$ on the triazole ring,

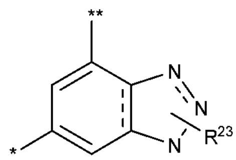

can be

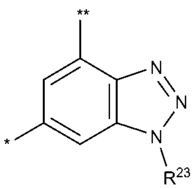 , 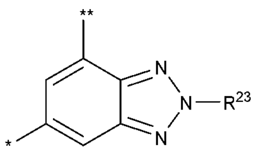 , or 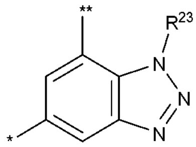 ;

wherein $R^{23}$ is selected from H or $C_{1-3}$ alkyl, * indicates represents the site connected to $CH_2$, ** represents the site connected to NH.

In one embodiment,

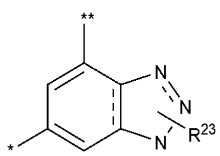

is

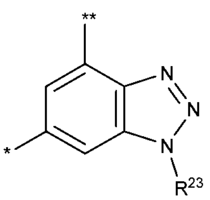 ,

wherein $R^{23}$ is methyl; * represents the site connected to $CH_2$, ** represents the site connected to NH.

In one embodiment, the compound is selected from the following compounds:

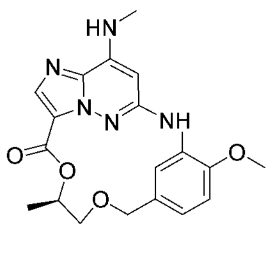
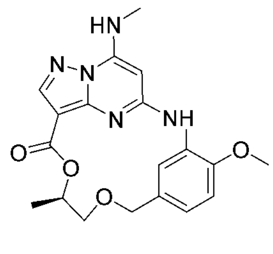
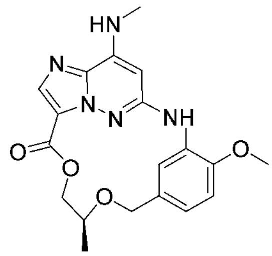

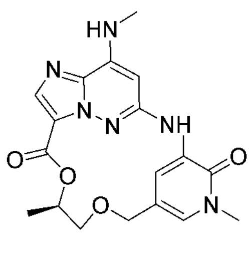
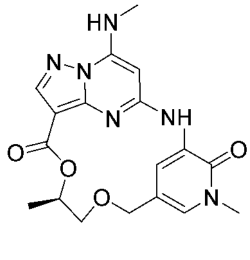
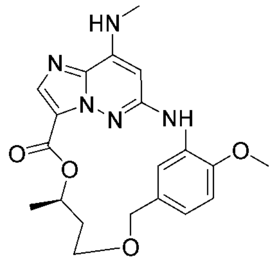

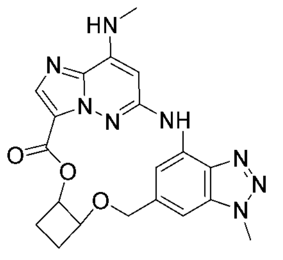
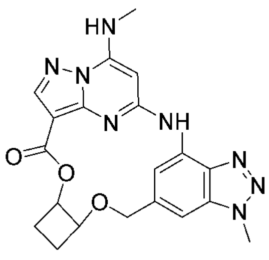
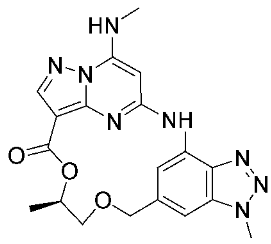

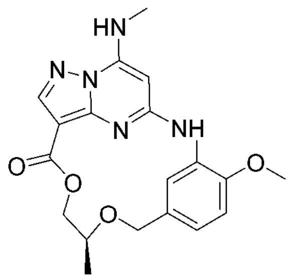
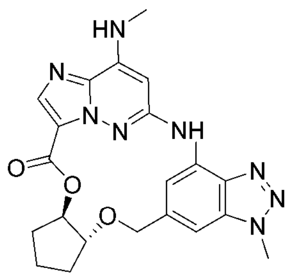
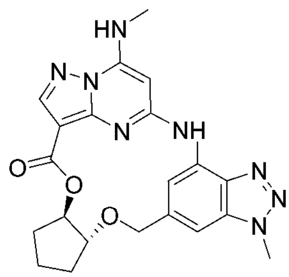

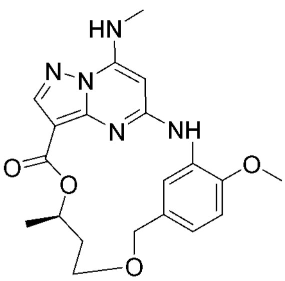
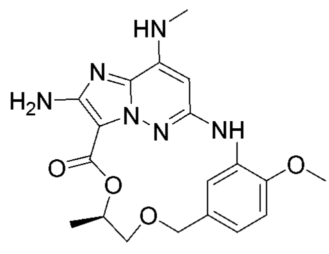
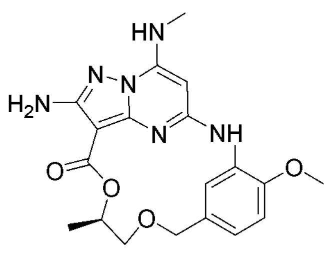

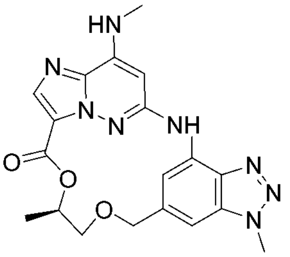
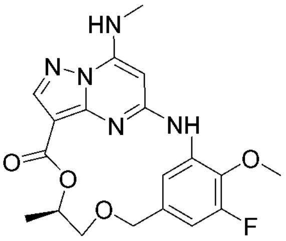
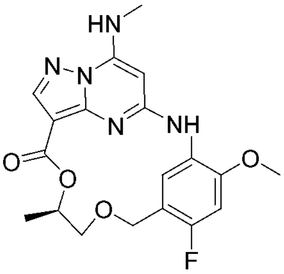
.

The compounds described in the present disclosure can be prepared by the following methods. The following methods and examples are to illustrate these methods. These procedures and examples should not be construed as limiting the present disclosure in any way. The compounds described herein can also be synthesized using standard synthesis techniques known to those skilled in the art, or methods known in the art and methods described herein can be used in combination.

The chemical reactions in the embodiments of the present disclosure are completed in a suitable solvent, and the solvent must be suitable for the chemical changes of the present disclosure and the reagents and materials required. In order to obtain the compounds of the present disclosure, it is sometimes necessary for those skilled in the art to modify or select the synthesis steps or reaction schemes based on the existing embodiments.

An important consideration in the planning of any synthetic route in the art is to select an appropriate protecting group for the reactive functional group (such as the amino group in the present disclosure). For trained practitioners, Greene and Wuts (Protective Groups In Organic Synthesis, Wiley and Sons, 1991) is the authority in this regard. All references cited in the present disclosure are incorporated into the present disclosure in their entirety.

The reactions described herein can be monitored according to any suitable method known in the art. For example, product formation can be monitored by a broad spectrum method such as nuclear magnetic resonance spectroscopy (such as $^{1}H$ or $^{13}C$), infrared spectroscopy, spectrophotometry (such as UV-visible light), mass spectrometry, etc., or chromatography such as high performance liquid chromatography (HPLC) or thin layer chromatography.

The compound shown in Formula I of the present disclosure can be prepared by those skilled in the field of organic synthesis using standard methods in the art through the following procedures:

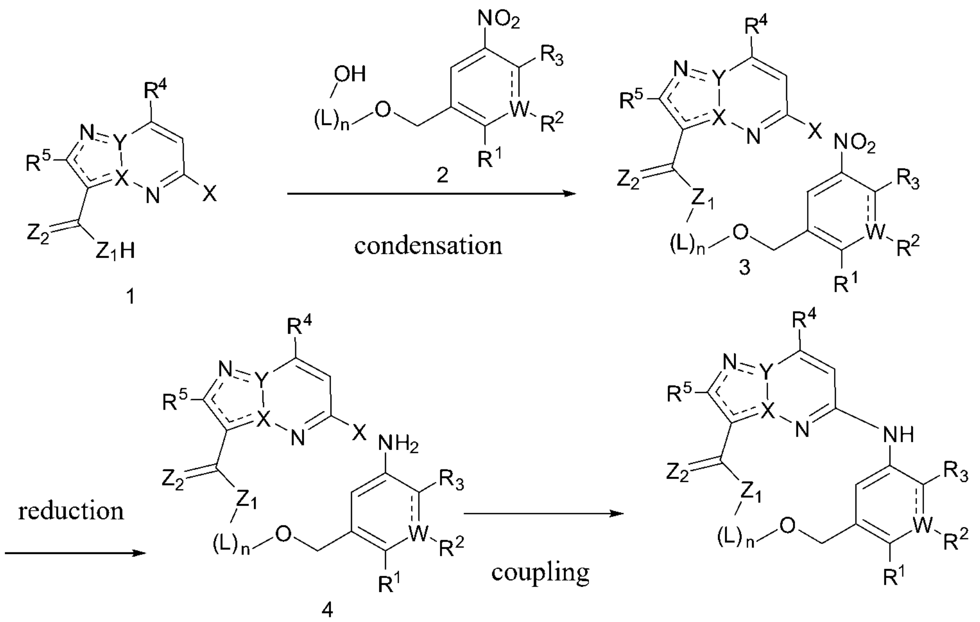


Formula I

Compound 1 and compound 2 undergo a condensation reaction to afford compound 3. Compound 3 is then subjected to a reduction reaction to yield compound 4. Subsequently, compound 4 undergoes an intramolecular coupling reaction to generate the compound of Formula I of the present disclosure.

Pharmaceutical composition and use thereof

The present disclosure provides a pharmaceutical composition comprising the compound as disclosed above, or pharmaceutically acceptable salts, hydrates, solvates, active metabolites, polymorphs, isotopic markers, isomers or prodrugs thereof, and a pharmaceutically acceptable carrier.

As demonstrated herein, the compound of the present disclosure exhibits excellent TYK2 inhibitory activity, particularly against the TYK2 JH2 pseudokinase domain, so that the it can be used for regulating TYK2-mediated diseases as a highly selective TYK2 allosteric inhibitors with strong blood-brain barrier penetration. Therefore, the present disclosure also provides use of the compound or pharmaceutically acceptable salts, solvates, active metabolites, polymorphs, isotopic markers, isomers or prodrugs thereof, as well as the pharmaceutical composition in preparation of pharmaceuticals for treating tyrosine kinase-mediated diseases.

In one embodiment, the tyrosine kinase is selected from TYK2 kinases. In one embodiment, the tyrosine kinase-mediated diseases include inflammatory autoimmune diseases, tumors, and neurodegenerative diseases and the like. The inflammatory autoimmune diseases mainly include atopic dermatitis, hidradenitis suppurativa, psoriasis, psoriatic arthritis, Crohn’s disease, ulcerative colitis, lupus erythematosus, and central nervous system autoimmune diseases such as autoimmune encephalopathies; the tumors mainly include leukemias, lymphomas, myelomas, and brain tumors; the neurodegenerative diseases mainly include cerebral atrophy, dementia, Parkinson’s syndrome, Alzheimer’s disease, amyotrophic lateral sclerosis, and multiple sclerosis; and the like.

The present disclosure further provides a method for treating tyrosine kinase-mediated diseases, comprising administering a therapeutically effective amount of the compound disclosed, or pharmaceutically acceptable salts, hydrates, solvates, active metabolites, polymorphs, isotopic markers, isomers or prodrugs thereof, or the pharmaceutical composition disclosed to a patient in need of administration.

The invention will be described in more detail with reference to the following examples. These examples are only provided for illustrative purposes and should not be construed as limiting the scope of the disclosure in any way. Those skilled in the art will readily recognize that various non-essential parameters may be altered or modified to obtain substantially the same results. According to the measurement(s) described herein, the compounds in the examples below have been identified as TYK2 allosteric inhibitors.

Example 1: (7*R*,*E*)-$3^6$-methoxy-7-methyl-$1^8$-(methylamino)-5,8-dioxa-2-aza-1(6,3)-imidazo[1,2-b]pyridazin-3(1,3)-benzocyclononan-9-one

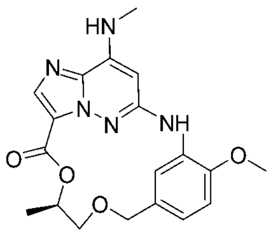

Synthesis route:

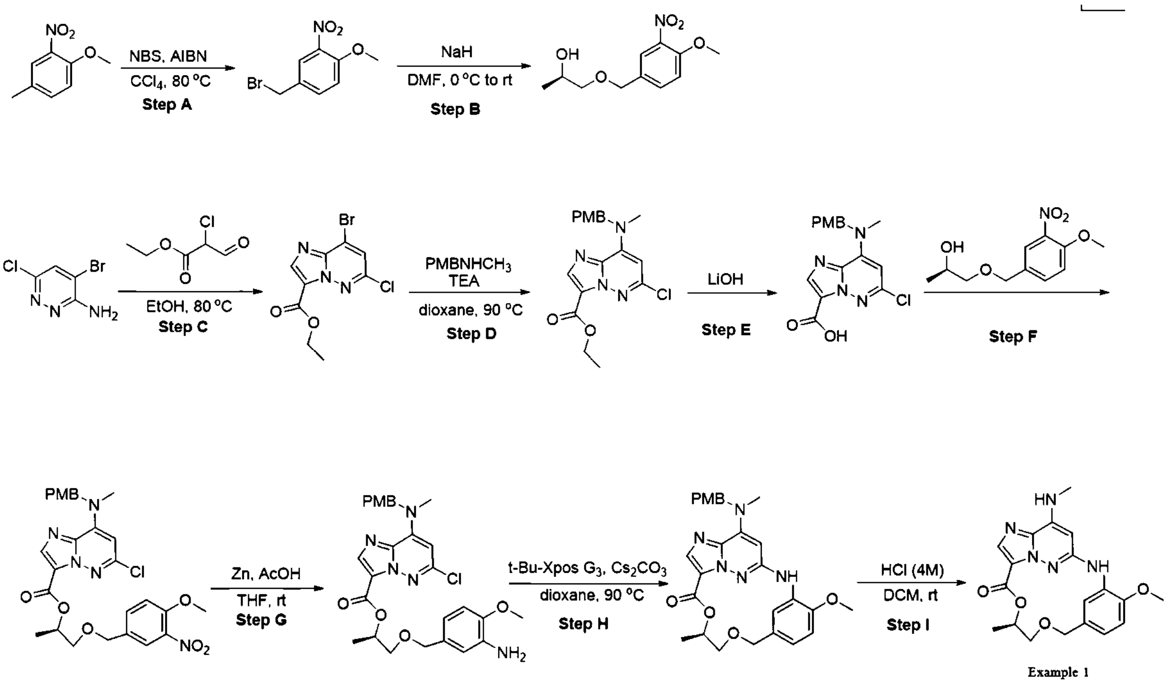


Example 1

Step A: 4-(bromomethyl)-1-methoxy-2-nitrobenzene

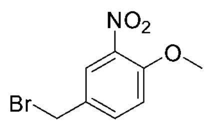

1-methoxy-4-methyl-2-nitrobenzene (10 g, 59.8 mmol, 1.0 eq) was added to carbon tetrachloride (150 mL), and then, after nitrogen purging, N-bromosuccinimide (10.7 g, 60.4 mmol, 1.01 eq) and azobisisobutyronitrile (1.96 g, 12.0 mmol, 0.2 eq) were added thereto. Subsequently, the reaction system was heated to 80 °C and subjected to the reaction overnight. After the reaction had been completed, the mixture was cooled to room temperature and concentrated, and the residue was diluted with water and extracted with dichloromethane. The combined organic phases were rinsed with water, dried over anhydrous sodium sulfate, and concentrated under reduced pressure. The residue was purified by silica gel column chromatography (PE:EA = 10:1 to 4:1) to give the product (13 g, yield = 88%).

Step B: (R)-1-((4-methoxy-3-nitrobenzyl)oxy)propan-2-ol

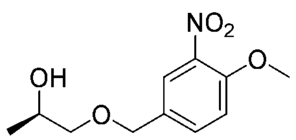

4-(bromomethyl)-1-methoxy-2-nitrobenzene (5 g, 20.3 mmol, 1.0 eq) was added to N,N-dimethylformamide (50 mL), and then (R)-1,2-propane diol (1.55 g, 20.3 mmol, 1.0 eq) was added. After nitrogen purging, the reaction system was cooled to 0 °C, and 60% sodium hydride (0.73 g, 30.5 mmol, 1.5 eq) was added thereto in batches. The mixture was then warmed to room temperature and subjected to the reaction for 2 hours. After the reaction had been completed and quenched with water under an ice bath, the pH value was adjusted to 6-7 with 3 M hydrochloric acid solution, and the mixture was extracted with ethyl acetate. The combined organic phases were rinsed with water, dried over anhydrous sodium sulfate, and concentrated under reduced pressure. The residue was purified by silica gel column chromatography (PE:EA = 5:1 to 4:1) to give the product (1.28 g, yield = 27%).
LC-MS: $(M+H)^+$; m/z=242.1

Step C: ethyl 8-bromo-6-chloroimidazo[1,2-b]pyridazine-3-carboxylate

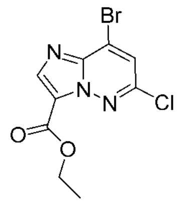

4-bromo-6-chloropyridazin-3-amine (20 g, 96.0 mmol, 1.0 eq) was added to ethanol (200 mL), and then ethyl 2-chloro-3-oxopropanoate (23.1 g, 153.5 mmol, 1.6 eq) was added. After nitrogen purging, the reaction system was heated to 80 °C and subjected to the reaction overnight. After the reaction had been completed, the solvent was removed under reduced pressure, and the residue was extracted with ethyl acetate. The combined organic phases were rinsed with water, dried over anhydrous sodium sulfate, and concentrated under reduced pressure. The residue was purified by silica gel column chromatography (PE:EA = 6:1 to 5:1) to give the product (21 g, yield = 72%).
LC-MS: $(M+H)^+$; m/z=303.9

Step D: Ethyl 6-chloro-8-((4-methoxybenzyl)(methyl)amino)imidazo[1,2-b]pyridazine-3-carboxylate

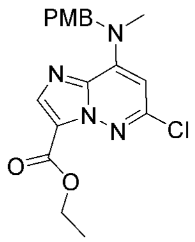

Ethyl 8-bromo-6-chloroimidazo[1,2-b]pyridazine-3-carboxylate (10 g, 32.8 mmol, 1.0 eq) was added to 1,4-dioxane (100 mL), and then [(4-methoxyphenyl)methyl](methyl)amine (5.46 g, 36.1 mmol, 1.1 eq) and triethylamine (6.65 g, 65.7 mmol, 2.0 eq) were added thereto. After nitrogen purging, the reaction system was heated to 90 °C and subjected to the reaction overnight. After the reaction had been completed, the mixture was cooled to room temperature and concentrated, and the residue was diluted with water and extracted with dichloromethane. The combined organic phases were rinsed with water, dried over anhydrous sodium sulfate, and concentrated under reduced pressure, to give the product (12 g, yield = 97%).
LC-MS: $(M+H)^+$; m/z=375.1

Step E: 6-chloro-8-((4-methoxybenzyl)(methyl)amino)imidazo[1,2-b]pyridazine-3-carboxylic acid

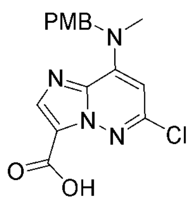

At room temperature, ethyl 6-chloro-8-((4-methoxybenzyl)(methyl)amino)imidazo[1,2-b]pyridazine-3-carboxylate (5 g, 13.3 mmol, 1.0 eq) was added to tetrahydrofuran (150 mL). Then, lithium hydroxide (1.28 g, 53.4 mmol, 4.0 eq) dissolved in 25 mL of water was added, and the mixture was stirred at room temperature overnight. The pH was adjusted to 4 with 1 M HCl solution. The aqueous phase was extracted with ethyl acetate. The combined organic phases were rinsed

with water, dried over anhydrous sodium sulfate, and concentrated under reduced pressure to give the crude product, which was used directly in the next step.
LC-MS: $(M+H)^+$; m/z=347.1

Step F: *(R)-1-((4-methoxy-3-nitrobenzyl)oxy)propan-2-yl-6-chloro-8-((4-*methoxybenzyl)(methyl)amino)imidazo[1,2-b]pyridazine-3-formate

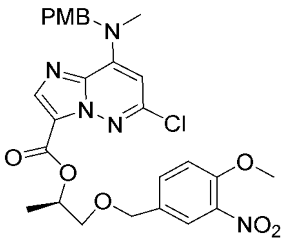

6-chloro-8-((4-methoxybenzyl)(methyl)amino)imidazo[1,2-b]pyridazine-3-carboxylic acid (600 mg, 1.7 mmol, 1.0 eq) was added to dichloromethane (6 mL), and then (R)-1-((4-methoxy-3-nitrobenzyl)oxy)propan-2-ol (835 mg, 3.5 mmol, 2.0 eq), dicyclohexylcarbodiimide (428 mg, 2.1 mmol, 1.2 eq), and N,N-4-dimethylaminopyridine (21 mg, 0.2 mmol, 0.1 eq) were added thereto. The mixture was stirred at room temperature overnight. The solvent was evaporated under reduced pressure, and the residue was extracted with dichloromethane. The combined organic phases were rinsed with water, dried over anhydrous sodium sulfate, and concentrated under reduced pressure. The residue was purified by silica gel column chromatography (PE:EA = 5:1 to 4:1) to give the product (1.1 g, yield = 89%).
LC-MS: $(M+H)^+$; m/z=570.2

Step G: (R)-1-((3-amino-4-methoxybenzyl)oxy)propan-2-yl-6-chloro-8-((4-methoxyphenyl)(methyl)amino)imidazo[1,2-b]pyridazine-3-formate

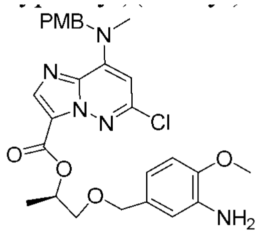

*(R)-1-((4-methoxy-3-nitrobenzyl)oxy)propan-2-yl-6-chloro-8-((4-*methoxybenzyl)(methyl)amino)imidazo[1,2-b]pyridazine-3-carboxylate (100 mg, 0.2 mmol, 1.0 eq) was added to tetrahydrofuran (1 mL), and then acetic acid (0.1 mL) and zinc powder (115mg, 1.8 mmol, 10 eq) were added thereto. The mixture was stirred at room temperature overnight. After the reaction had been completed, the solvent was evaporated under reduced pressure, and the residue was extracted with ethyl acetate. The combined organic phases were rinsed with water, dried over anhydrous sodium sulfate, and concentrated under reduced pressure, to give the crude product, which was used directly in the next step.
LC-MS: $(M+H)^+$; m/z=540.2

Step H: (7*R*,*E*)-$3^6$-methoxy-$1^8$-((4-methoxybenzyl)(methyl)amino)-7-methyl-5,8-dioxa-2-aza-1(6, 3)-imidazo[1,2-b]pyridazin-3(1,3)-benzheterocyclononan-9-one

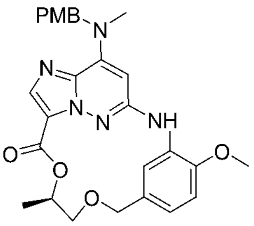

(R)-1-((3-amino-4-methoxybenzyl)oxy)propan-2-yl-6-chloro-8-((4-methoxyphenyl)(methyl)amino)imidazo[1,2-b]pyridazine-3-carboxylate (900 mg, crude), 1,4-dioxane (5 mL), cesium carbonate (869 mg, 2.7 mmol, 2.0 eq), di-tert-butyl[2, 4, 6-tri(propyl-2-yl)-[1,1'-biphenyl]-2-yl]phosphine (106 mg, 0.1 mmol, 0.1 eq), and 2'-amino-[1,1'-biphenyl]-2-yl} palladium methanesulfonate were added to a 10 mL reaction vial at room temperature. The atmosphere was immediately purged with nitrogen, and the mixture in the reaction vial was stirred at 90°C overnight. After cooled, the reaction mixture was concentrated, diluted with water, and extracted with ethyl acetate. The organic phases were combined and concentrated, and the residue was purified by column chromatography to give the product (170 mg, 25%).
LC-MS: $(M+H)^+$; m/z=504.2

Step I: (7*R*,*E*)-$3^6$-Methoxy-7-methyl-$1^8$-(methylamino)-5,8-dioxa-2-aza-1(6,3)-imidazo[1,2-b]pyridazin-3(1,3)-benzheterocyclononan-9-one

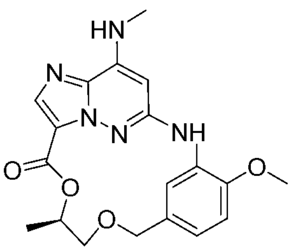

(7*R*,*E*)-$3^6$-methoxy-$1^8$-((4-methoxybenzyl)(methyl)amino)-7-methyl-5,8-dioxa-2-aza-1(6,3)-imidazo[1,2-b]pyridazin-3(1,3)-benzheterocyclononan-9-one (160 mg, 0.3 mmol, 1.0 eq) was dissolved in dichloromethane (3 mL), followed by adding hydrochloric acid (1 ml, 4 M in 1,4-dioxane) solution and stirring at room temperature for two hours. The reaction mixture was concentrated, and the residue was purified by high performance liquid chromatography to give the product (8.8 mg).

LC-MS: $(M+H)^+$; m/z=384.2
$^1$H-NMR (400 MHz, DMSO-d6) δ 9.05 (s, 1H), 8.21 (s, 1H), 8.00 (s, 1H), 7.26 (d, *J*=5.2 Hz, 1H), 6.95 (d, J=8.0 Hz, 1H), 6.70 (d, J=8.0, 1H), 6.33 (s, 1H), 5.07-5.06 (m, 1H), 4.63-4.51 (m, 2H), 388 (s, 3H), 3.68-3.60 (m, 2H), 3.32 (s, 1H), 2.87 (d, *J*=4.8 Hz, 3H), 1.32 (d, *J*=6.4 Hz, 3H).

Example 2: (*R*,$1^3$*E*,$1^4$*E*)-$3^6$-methoxy-7-methyl-$1^7$-(methylamino)-5,8-dioxa-2-aza-1(5,3)-pyrazolo[1,5-a]pyrimidin-3(1,3)-benzheterocyclononan-9-one

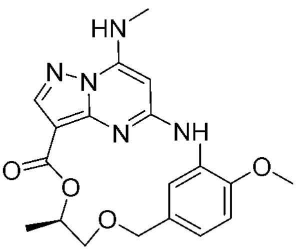

Synthesis route:

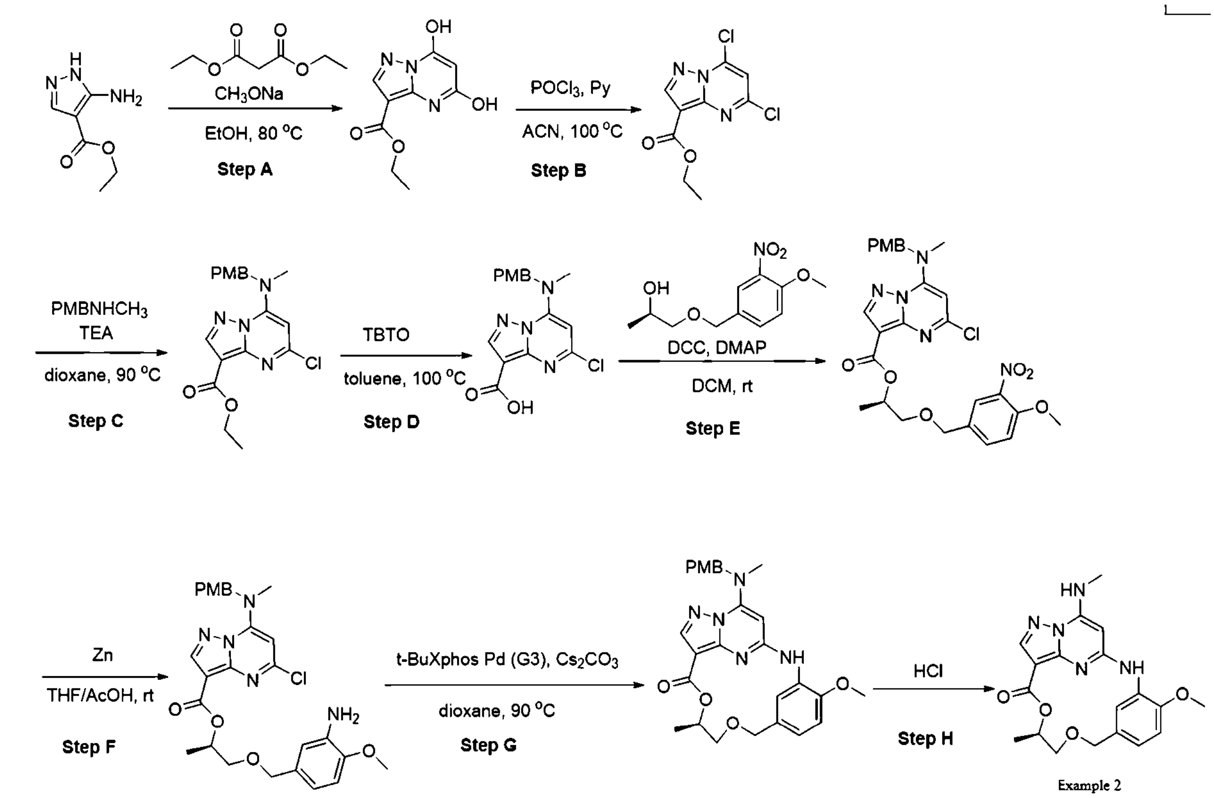


Example 2

Step A: Ethyl 5,7-dihydroxypyrazolo[1,5-a]pyrimidine-3-carboxylate

Ethyl 5-amino-1H-pyrazole-4-carboxylate (20.0 g, 129.0 mmol, 1.0 eq) was dissolved in ethanol (200 mL) at room temperature, and then sodium methoxide (21.3 g, 387.1 mmol, 3.0 eq) and dimethyl malonate (31.0 g, 194.1 mmol, 1.5 eq) were added sequentially. The reaction mixture was stirred at 80°C overnight. After the reaction had been completed, the solvent was removed by evaporation under reduced pressure, and the residue was diluted with water to precipitate a solid, which was collected by filtration and dried to give the product (21.0 g, 73%).
LC-MS: $(M+H)^+$; m/z = 224.1

Step B: Ethyl 5,7-dichloropyrazolo[1,5-a]pyrimidine-3-carboxylate

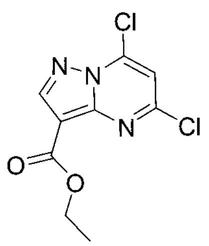

Ethyl 5,7-dihydroxypyrazolo[1,5-a]pyrimidine-3-carboxylate (21.0 g, 93.8 mmol, 1.0 eq) was dissolved in acetonitrile (200 mL) at room temperature, and then pyridine (22.2 g, 281.3 mmol, 3.0 eq) and phosphorus oxychloride (73.0 g, 469.1 mmol, 5.0 eq) were added sequentially. The reaction mixture was stirred at 100°C overnight. After the reaction had been completed, the phosphorus oxychloride was removed by concentration under reduced pressure. The residue was diluted with water under an ice bath, and the pH was adjusted to neutral with saturated sodium bicarbonate

solution. The mixture was extracted with dichloromethane and concentrated under reduced pressure to give the product (17.0 g, 70%).
LC-MS: $(M+H)^+$; m/z = 260.0

Step C: ethyl 5-chloro-7-((4-methoxybenzyl)(methyl)amino)pyrazolo[1,5-a]pyrimidine-3-carboxylate

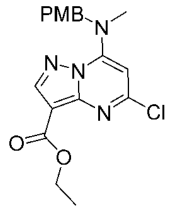

Ethyl 5,7-dichloropyrazolo[1,5-a]pyrimidine-3-carboxylate (5 g, 19.2 mmol, 1.0 eq) was added to 1,4-dioxane (100 mL), followed by [(4-methoxyphenyl)methyl](methyl)amine (5.19 g, 21.1 mmol, 1.1 eq) and triethylamine (3.89 g, 38.4 mmol, 2.0 eq). After nitrogen purging, the reaction system was heated to 90°C and subjected to the reaction overnight. After the reaction had been completed, the mixture was cooled to room temperature, concentrated, diluted with water, and extracted with dichloromethane. The combined organic phases were rinsed with water, and dried over anhydrous sodium sulfate. The residue was purified by column chromatography to give the product (2.1 g, 30%).
LC-MS: $(M+H)^+$; m/z = 375.1

Step D: 5-chloro-7-[(4-methoxyphenyl)methyl](methyl)amino}pyrazolo[1,5-a]pyrimidine-3-carboxylic acid

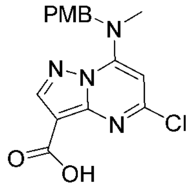

Ethyl 5-chloro-7-((4-methoxybenzyl)(methyl)amino)pyrazolo[1,5-a]pyrimidine-3-carboxylate (2.0 g, 5.3 mmol, 1.0 eq) was added to toluene (20 mL), followed by tributyltin oxide (4.7 g, 8.0 mmol, 1.5 eq). The reaction mixture was subjected to the reaction at 100°C overnight. After the reaction had been completed, the mixture was cooled to room temperature, concentrated, diluted with water, and extracted with ethyl acetate. The combined organic phases were rinsed with water and dried over anhydrous sodium sulfate. The residue was purified by reverse-phase C18 column to give the product (1.0 g, 54%).
LC-MS: $(M+H)^+$; m/z = 347.1

Step E: (R)-1-((4-methoxy-3-nitrobenzyl)oxy)propan-2-yl-5-chloro-7-((4-methoxybenzyl)(methyl)amino)pyrazolo[1,5-a]pyrimidine-3-carboxylate

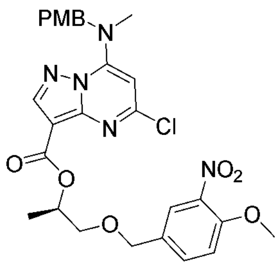

5-chloro-7-[(4-methoxyphenyl)methyl](methyl)amino}pyrazolo[1,5-a]pyrimidine-3-carboxylic acid (1 g, 2.9

mmol, 1.0 eq) was dissolved in dichloromethane (20 mL) at room temperature. Then, (2R)-1-[(4-methoxy-3-nitrophenyl) methoxy]propanol (1.39 g, 5.8 mmol, 2.0 eq), N,N'-dicyclohexylcarbodiimide (0.71 g, 3.5 mmol, 1.2 eq), and 4-dimethylaminopyridine(40 mg, 0.3 mmol, 0.1 eq) were added sequentially. The reaction mixture was stirred at room temperature overnight. After the reaction had been completed, the mixture was diluted with water and extracted with dichloromethane. The organic phases were combined, dried over sodium sulfate, and concentrated under reduced pressure. The residue was purified by column chromatography to give the product as a light yellow oil (1.5 g, 73%).
LC-MS: $(M+H)^+$; m/z = 570.2

Step F: (R)-1-((3-amino-4-methoxybenzyl)oxy)propan-2-yl-5-chloro-7-((4-methoxyphenyl)(methyl)amino)pyrazolo[1,5-a]pyrimidine-3-carboxylate

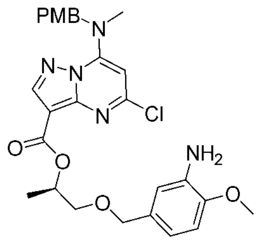

*(R)-1-((4-methoxy-3-nitrobenzyl)oxy)propan-2-yl-5-chloro-7-((4*-methoxybenzyl)(methyl)amino)pyrazolo[1,5-a]pyrimidine-3-carboxylate (1.4 g, 2.0 mmol, 1.0 eq)was dissolved in tetrahydrofuran (20 mL) at room temperature. Then, acetic acid (2 mL) and zinc powder (1.28 g, 19.7 mmol, 10 eq) were added sequentially. The reaction mixture was stirred at room temperature overnight. After the reaction had been completed, the zinc powder was removed by filtration. The filtrate was diluted with water and extracted with ethyl acetate. The combined organic phases were rinsed with water, dried over sodium sulfate, and concentrated under reduced pressure to give the product (1.4 g, crude product).
LC-MS: $(M+H)^+$; m/z = 540.2

Step G: (*R*,$1^3 5^4$*E*)-$3^6$-methoxy-$1^7$-((4-methoxybenzyl)(methyl)amino)-7-methyl-5,8-dioxa-2-aza-1(5,3)-pyrazolo[1,5-a] pyrimidin-3(1,3)-benzheterocyclononan-9-one

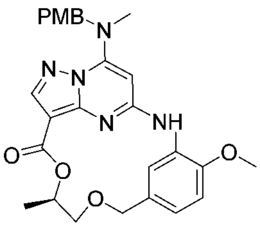

*(R)-1-((3-amino-4-methoxybenzyl)oxy)propan-2-yl-5-chloro-7-((4*-methoxyphenyl)(methyl)amino)pyrazolo [1,5-a]pyrimidine-3-carboxylate (1.3 g, crude) was dissolved in 1,4-dioxane (15 mL) at room temperature. Then, cesium carbonate (1.18 g, 3.6 mmol, 2.0 eq), di-tert-butyl[2, 4, 6-tri(propyl-2-yl)-[1,1'-biphenyl]-2-yl]phosphine (140 mg, 0.2 mmol, 0.1 eq), and 2'-amino-[1,1'-biphenyl]-2-yl}methanesulfonic acid palladium ester were added sequentially. After nitrogen purging, the reaction system was stirred at 90°C for 6 hours. The mixture was cooled and concentrated under reduced pressure. The residue was diluted with water and extracted with ethyl acetate. The combined organic phases were rinsed with water, dried over anhydrous sodium sulfate, and concentrated under reduced pressure. The residue was purified by column chromatography to give a light yellow solid product (750 mg, 74%).
LC-MS: $(M+H)^+$; m/z = 504.2

Step H: (*R*,$1^3$*E*$^4$*E*)-$3^6$-methoxy-7-methyl-$1^7$-(methylamino)-5,8-dioxa-2-azazo-1(5,3)-pyrazolo[1,5-a]pyrimidine-3(1,3)-benzheterocyclononan-9-one

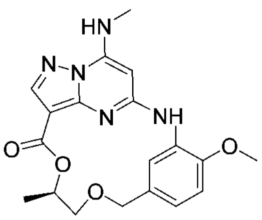

(*R*, $1^3E$, $1^4E$)-$3^6$-methoxy-$1^7$-((4-methoxybenzyl)(methyl)amino)-7-methyl-5,8-dioxa-2-aza-1(5,3)-pyrazolo[1,5-a]pyrimidin-3(1,3)-benzheterocyclononan-9-one (650 mg, 1.3 mmol, 1.0 eq) was dissolved in dichloromethane (10 mL), and then 4M HCl in 1,4-dioxane (3mL) was added. The mixture was stirred at room temperature for 2 hours. After the reaction had been completed, the mixture was concentrated under reduced pressure. The crude product was purified by high performance liquid chromatography to give the product (96.8 mg, 20%).

LC-MS: $(M+H)^+$; m/z = 384.2
$^1$H-NMR (400 MHz, DMSO-*d*6) δ 9.37 (s, 1H), 8.71 (s, 1H), 8.23 (s, 1H), 7.70 (d, *J*=4.8Hz, 1H), 6.95 (d, *J*=8.4Hz, 1H), 6.75 (d, *J*=8.0Hz, 1H), 6.06 (s, 1H), 4.87 (t, *J*=5.2Hz, 1H), 4.60-4.52 (m, 2H), 3.87 (s, 3H), 3.66- 3.61 (m, 2H), 3.32 (s, 2H), 2.90 (d, *J*=4.8Hz, 3H), 1.32 (d, *J*=6.4Hz, 3H).

The following examples were prepared according to the experimental route and method in Example 1 or 2:

| | | |
|---|---|---|
| Example 3: (6*S*, *E*)-$3^6$-methoxy-6-methyl-$1^8$-(methylamino)-5,8-dioxa-2-azazo-1(6,3)-imidazo[1,2-b]pyridazine-3(1,3)-benzheterocyclononan-9-one | | |
| 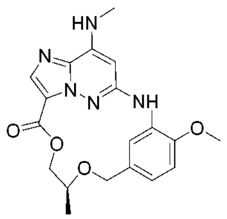 | LC-MS: $(M+H)^+$; m/z=384.3<br>$^1$H-NMR (400 MHz, DMSO-*d*6) δ 9.13 (s, 1H), 8.21 (s, 1H), 8.03 (s, 1H), 7.27 (d, *J*=5.1Hz, 1H), 6.94 (d, *J*=8.2 Hz, 1H), 6.71 (d, *J*=7.6Hz, 1H), 6.34 (s, 1H), 4.67-4.55 (m, 2H), 4.28-4.25 (m, 1H), 4.08 (t, *J*=9.6Hz, 1H), 3.97-3.93 (m, 1H), 3.88 (s, 3H), 2.87 (d, *J*=4.4Hz, 3H), 1.24 (s, 1H), 1.17 (d, *J*=5.6Hz, 3H). | according to Example 1 |
| Example 4: *(S,* $1^3E$,$1^4E$)-$3^6$-methoxy-6-methyl-$1^7$-(methylamino)-5,8-dioxa-2-azazo-1(5,3)-pyrazolo[1,5-a]pyrimidine-3(1,3)-benzheterocyclononan-9-one | | |
| 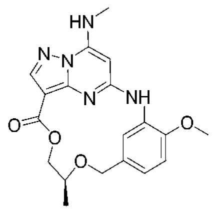 | LC-MS: $(M+H)^+$; m/z=384.2<br>$^1$H-NMR (400 MHz, DMSO-d6) δ 9.54 (s, 1H), 8.72 (s, 1H), 8.26 (s, 1H), 7.72 (d, *J*=5.2Hz, 1H), 6.93 (d, *J*=8.0Hz, 1H), 6.76 (d, *J*=8.4Hz, 1H), 6.08 (s, 1H), 4.68-4.50 (m, 2H), 4.22 (d, *J*=10.8Hz, 1H), 3.91-3.87 (m, 5H), 2.90 (d, *J*=4.4Hz, 3H), 1.24 (s, 1H), 1.16 (d, *J*=5.6Hz, 3H). | according to Example 2 |
| Example 5: ($6^1R$,$6^2R$,*E*)-$3^1$-methyl-$1^8$-(methylamino)-$3^1$H-5,7-dioxa-2-azazo-1(6,3)-imidazo[1,2-b]pyridazine-3(4,6)-benzo[d][1,2,3]triazole-6(1,2)-cyclopentane cyclooctafuran-8-one | | |
| 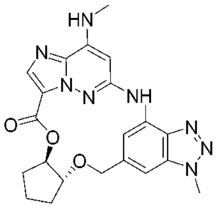 | LC-MS: $(M+H)^+$; m/z=435.1<br>$^1$H-NMR (400 MHz, DMSO-$d_6$) δ 9.76 (s, 1H), 8.95 (s, 1H), 8.05 (s, 1H), 7.41 (d, *J*= 4.8Hz, 1H), 7.11 (s, 1H), 6.56 (s, 1H), 5.00-4.96 (m, 1H), 4.92-4.86 (m, 1H), 4.75-4.71 (m, 1H), 4.26-4.23 (m, 4H), 4.30-4.19 (m, 1H), 2.89 (d, *J*=4.8Hz, 3H), 2.41-2.35 (m, 1H), 2.19-2.08 (m, 1H), 1.78-1.73 (m, 1H), 1.67-1.60 (m, 1H), 1.54-1.41 (m, 2H). | according to Example 1 |
| Example 6: ($1^3E$,$1^4E$,$6^1R$,$6^2R$)-$3^1$-methyl-$1^7$-(methylamino)-$3^1$H-5,7-dioxa-2-azazo-1(5,3)-pyrazolo[1,5-a]pyrimidine-3(4,6)-benzo[d][1,2,3]triazole-6(1,2)-cyclopentane cyclooctafuran-8-one | | |

(continued)

| | | |
|---|---|---|
| 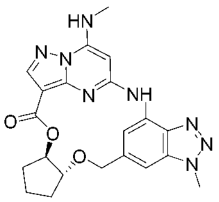 | LC-MS: $(M+H)^+$; m/z=435.1<br>$^1$H-NMR (400MHz, $CDCl_3$) δ 9.52 (s, 1H), 8.35 (s, 1H), 8.15 (s, 1H), 6.89 (s, 1H), 6.23 (d, *J*=4.8 Hz, 1H), 5.61 (s, 1H), 5.11 (d, *J*=14.2Hz, 1H), 4.99-4.93 (m, 1H), 4.77 (d, *J*=14.2Hz, 1H), 4.49-4.35 (m, 1H), 4.29 (s, 3H), 3.14 (d, *J* = 4.8Hz, 3H), 2.66-2.64 (m, 1H), 2.20-2.17 (m, 1H), 1.88-1.72 (m, 1H), 1.69-1.64 (m, 2H), 1.28-1.20 (m, 1H). | according to Example 2 |
| Example 7: ($1^5E$,$3^3E$,7*R*)-$3^1$,7-dimethyl-$1^8$-(methylamino)-$3^1$,$3^2$-dihydro-5,8-dioxa-2-azazo-1(6,3)-imidazo[1,2-b] pyridazine-3(3,5)-pyridine heterocyclononane-$3^2$,9-dione | | |
| 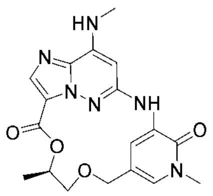 | LC-MS: $(M+H)^+$; m/z=385.1 | according to Example 1 |
| Example 8: (*R*,$1^3E$,$1^4E$,$3^3E$)-$3^1$,7-dimethyl-$1^7$-(methylamino)-$3^1$,$3^2$-dihydro-5,8-dioxa-2-azazo-1(5, 3)-pyrazolo[1,5-a]pyrimidine-3 (3,5)-pyridine heterocyclononane-$3^2$, 9-dione | | |
| 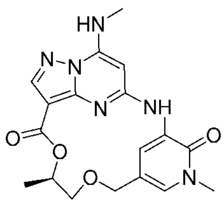 | LC-MS: $(M+H)^+$; m/z=385.1 | according to Example 2 |
| Example 9: (8*R*,*E*)-$3^6$-methoxy-8-methyl-$1^8$-(methylamino)-5,9-dioxa-2-azazo-1(6,3)-imidazo[1,2-*b*]pyridazine-3(1,3)-benzheterocyclodecane-10-one | | |
| 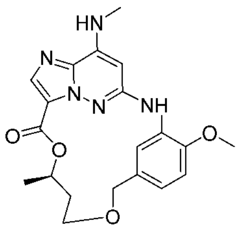 | LC-MS: $(M+H)^+$; m/z=398.1 | according to Example 1 |
| Example 10: (*R*,$1^3E$,$1^4E$)-$3^6$-methoxy-8-methyl-$1^7$-(methylamino)-5,9-dioxa-2-azazo-1(5,3)-pyrazolo[1,5-*a*]pyrimidine-3(1,3)-benzheterocyclodecane-10-one | | |
| 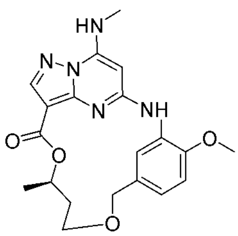 | LC-MS: $(M+H)^+$; m/z=398.1 | according to Example 2 |
| Example 11: (7R,E)-$1^2$-amino-$3^6$-methoxy-7-methyl-$1^8$-(methylamino)-5,8-dioxa-2-azazo-1(6,3)-imidazo[1,2-b]pyridazine-3(1,3)-benzheterocyclononan-9-one | | |
| 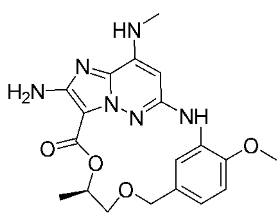 | LC-MS: $(M+H)^+$; m/z=399.1 | according to Example 1 |

(continued)

| | | |
|---|---|---|
| Example 12: (*R*,$1^3$*E*,$1^4$*E*)-$1^2$-amino-$3^6$-methoxy-7-methyl-$1^7$-(methylamino)-5,8-dioxa-2-azazo-1(5,3)-pyrazolo[1,5-a]pyrimidine-3(1,3)-benzheterocyclononan-9-one | | |
| 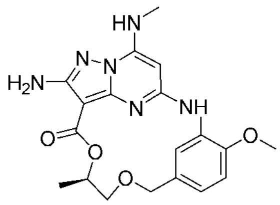 | LC-MS: (M+H)$^+$; m/z=399.1 | according to Example 2 |
| Example 13: (*E*)-$3^1$-methyl-$1^8$-(methylamino)-$3^1$H-5,7-dioxa-2-azazo-1(6,3)-imidazo[1,2-b]pyridazine-3(4,6)-benzo[d][1,2,3]triazole-6(1,2)-cyclobutane cyclooctafuran-8-one | | |
| 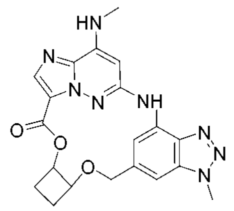 | LC-MS: (M+H)$^+$; m/z=421.1 | according to Example 1 |
| Example 14: ($1^3$ *E*,$1^4$*E*)-$3^1$-methyl-$1^7$ -(methylamino)-$3^1$H-5,7-dioxa-2-azazo-1(5,3)-pyrazolo[1,5-a]pyrimidine-3(4,6)-benzo[d][1,2,3]triazole-6(1,2)-cyclobutane cyclooctafuran-8-one | | |
| 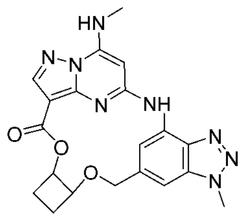 | LC-MS: (M+H)$^+$; m/z=421.1 | according to Example 2 |
| Example 15: (*R*,$1^3$*E*,$1^4$*E*)-$3^1$,7-dimethyl-$1^7$-(methylamino)-$3^1$*H*-5,8-dioxa-2-azazo-1(5,3)-pyrazolo[1,5-a]pyrimidine-3(4,6)-benzo[d][1,2,3]triazole heterocyclononan-9-one | | |
| 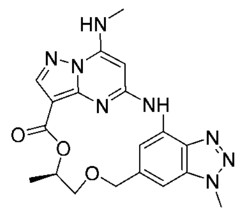 | LC-MS: (M+H)$^+$; m/z=409.1 | according to Example 2 |
| Example 16: (7*R*,*E*)-$3^1$,7-dimethyl-$1^8$-(methylamino)-$3^1$*H*-5,8-dioxa-2-azazo-1(6,3)-imidazo[1, 2-b]pyridazine-3(4,6)-benzo[d][1,2,3]triazole heterocyclononan-9-one | | |
| 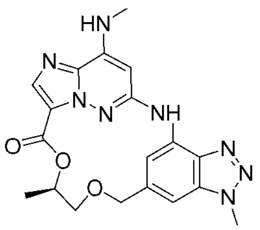 | LC-MS: (M+H)$^+$; m/z=409.1 | according to Example 1 |
| Example 17: (*R*,$1^3$*E*,$1^4$*E*)-$3^5$-fluoro-$3^6$-methoxy-7-methyl-$1^7$-(methylamino)-5,8-dioxa-2-azazo-1(5,3)-pyrazolo[1,5-a]pyrimidine-3(1,3)-benzheterocyclononan-9-one | | |

(continued)

| | | |
|---|---|---|
| 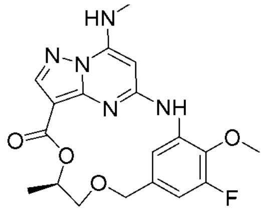 | LC-MS: $(M+H)^+$; m/z=402.1 | according to Example 2 |
| Example 18: (*R*,1[3]*E*,1[4]*E*)-3[4]-fluoro-3[6]-methoxy-7-methyl-1[7]-(methylamino )-5,8-dioxa-2-azazo-1(5,3)-pyrazolo[1,5-a]pyrimidine-3(1,3)-benzheterocyclononan-9-one | | |
| 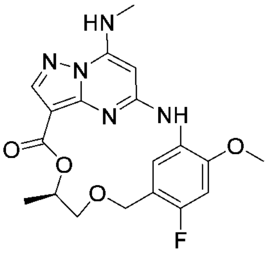 | LC-MS: $(M+H)^+$; m/z=402.1 | according to Example 2 |

Biological activity and pharmacokinetic experiments

1. Enzymatic activity($IC_{50}$) assay of compounds against TYK2-JH2 and JAK1-JH2

Reference compound A, Deucravacitinib, and the compounds to be tested were diluted from 10 mmol/L stock solutions to 0.2 mmol/L. Specifically, 1.2 μL of compound stock was added to 58.8 μL of DMSO, followed by 4-fold serial dilutions to generate 10 concentrations. Using an Echo liquid handler, 50 nL of the compound to be tested was transferred into a 384-well reaction plate, in duplicate wells for each compound. The plate was subjected to centrifugation at 1000 rpm for 1 min and the final DMSO concentration was 0.5%. Subsequently, 5 μL of TYK2 (or JAK1) was added to the plate, centrifuged at 1000 rpm for 1 min, and incubated at 25 °C for 10 min. Then, 5 μL of JH2 probe 1 was added to the plate, centrifuged at 1000 rpm for 1 min, and incubation at 25 °C for 60 min. FP 520/48 signals were measured using a BMG high-throughput multifunctional plate reader.
Data from the TYK2-JH2 assay were analyzed using GraphPad Prism 8 software. The readout of the negative control wells (0.5% DMSO) was defined as 0% inhibition, and the readout of the positive control wells (reference compound at maximum concentration) was defined as 100% inhibition. After calculating inhibition rates, $IC_{50}$ values (half-maximal inhibitory concentrations) of the control compounds and the compounds to be tested were determined using nonlinear regression fitting of software.

$$\%\ \text{inhibition rate of compound}=\left|\frac{\overline{\text{Data}}_{\text{negative control}}\text{-}\text{Data}_{\text{compound}}}{\overline{\text{Data}}_{\text{negative control}}\text{-}\overline{\text{Data}}_{\text{positive control}}}\right|*100\%$$

$\overline{\text{Data}}_{\text{positive control}}$: mean value of positive control wells
$\overline{\text{Data}}_{\text{negative control}}$: mean value of negative control wells

The specific $IC_{50}$ test results are shown in Table 1 below:

Table 1: $IC_{50}$ test results of the compounds in Examples

| Example | TYK2-JH2 $IC_{50}$ (nM) | JAK1-JH2 $IC_{50}$ (nM) |
|---|---|---|
| 1 | <5 | >5000 |
| 2 | <5 | >5000 |
| 3 | <20 | >5000 |
| 4 | <20 | >5000 |
| 5 | <5 | >5000 |

(continued)

| Example | TYK2-JH2 $IC_{50}$ (nM) | JAK1-JH2 $IC_{50}$ (nM) |
|---|---|---|
| 6 | <10 | |
| 7 | <5 | >5000 |
| 8 | <5 | >5000 |
| reference compound A | <5 | >1000 |
| Deucravacitinib | <5 | <5 |

From the data in the table, it can be seen that all the compounds in the examples according to the present disclosure exhibit excellent enzymatic inhibitory activity against TYK2-JH2, and some of them even have the $IC_{50}$ values below 5 nM. In contrast, the compounds show weak inhibition against JAK1-JH2 with $IC_{50}$ values greater than 5000 nM. Accordingly, it is believed that the compounds according to the present disclosure are powerful and highly selective TYK2 (tyrosine kinase 2) allosteric inhibitors.

2. Affinity assay ($K_D$) of compounds against JAK1-JH2 and TYK2-JH2

1) Buffer preparation: prepare an appropriate running buffer according to experimental requirements;
2) Pre-concentration: optimize immobilization conditions for TYK2/JAK1 using a CM5 chip for ligand immobilization;
3) Ligand immobilization: immobilize an appropriate amount of TYK2/JAK1 on the chip;
4) Kinetic measurement: select a suitable kinetic method, typically single-cycle kinetics or multi-cycle kinetics, set contact time, dissociation time, and flow rate according to experimental needs, apply a concentration gradient of the analyte based on the expected KD values, and record the molecular interaction in real time.
5) Data analysis: analyze the experimental data for TYK2 using Biacore Insight Evaluation Software, which will automatically perform affinity $K_D$ values fitting for TYK2-JH2 and JAK1-JH2.

It can be seen from the results that the compounds in the examples demonstrated high affinity against TYK2-JH2 and low affinity against JAK1-JH2, indicating exceptionally high selectivity for TYK2-JH2 over JAK1-JH2.

3. Pharmacokinetic experiment

Male SD rats were divided into groups of three animals each, which were administered the compound of Example 2 by intravenous injection (2 mg/kg), administered the compound of Example 2 by single oral gavage (10 mg/kg), and administered a control compound by single oral gavage (10 mg/kg), respectively. Animals were fasted overnight prior to dosing, beginning 10 hours before administration and continuing until 4 hours after dosing. For the intravenous group, blood samples were collected at 0.0833, 0.25, 0.5, 1, 2, 4, 6, 8, and 24 h post-dose. For the oral administration groups, blood samples were collected at 0.25, 0.5, 1, 2, 4, 6, 8, and 24 h post-dose. Blood samples (0.3 mL) were collected from the retro-orbital venous plexus under isoflurane anesthesia into heparinized tubes. The samples were centrifuged at 4000 rpm for 5 min at 4 °C, and plasma was transferred to centrifuge tubes and stored at -80°C until analysis. Plasma samples were processed by protein precipitation extraction, and the extracts were analyzed by LC/MS/MS. Pharmacokinetic results are shown in Tables 2 and 3:

Table 2: Pharmacokinetic parameters of the compound in Example 2 in rat plasma after intravenous administration

| PK parameters | Example 2 |
|---|---|
| $C_0$ (ng/mL) | 2260 |
| $T_{1/2}$ (hr) | 0.93 |
| $AUC_{0-t}$ (ng·hr/mL) | 2019 |
| $AUC_{0-\infty}$ (ng·hr/mL) | 2026 |
| Vz (L/Kg) | 1.33 |
| Cl (mL/min/kg) | 16.7 |
| Vss (L/Kg) | 1.28 |
| MRT (hr) | 1.28 |

Table 3: Pharmacokinetic parameters of the compound in Example 2 in rat plasma after oral administration

| PK parameters | Example 2 | Control compound A | Control compound B |
|---|---|---|---|
| $T_{1/2}$ (hr) | 2.46 | 3.07 | 1.08 |
| $T_{max}$ (hr) | 0.5 | 1.83 | 0.50 |
| $C_{max}$ (ng/mL) | 2457 | 126 | 192 |
| $AUC_{0-t}$ (ng·hr/mL) | 5774 | 592 | 327 |
| $AUC_{0-\infty}$ (ng·hr/mL) | 5785 | 735 | 332 |
| MRT (hr) | 1.97 | 3.16 | 1.63 |
| Bioavialibility (%) | 57.1% | | |

It can be seen from the data in the tables that the compound in Example 2 according to the present disclosure has good bioavailability of 57.1% via oral administration, and its oral drug exposure is much better than that of the control compounds A and B.

4. Blood-brain penetration test

Male SD rats were divided into groups of three animals each, which were administered the compounds to be tested either by single oral gavage (PO, 10 mg/kg) or by single intravenous injection (IV, 2 mg/kg). Animals were fasted overnight prior to dosing, beginning 10 hours before administration and continuing until 4 hours after dosing. Rats were sacrificed at 0.5 hours after PO administration (or 5 min after IV administration). Blood and brain tissues were collected. The samples were centrifuged at 4000 rpm for 5 min at 4 °C, and plasma was transferred to centrifuge tubes and stored at -80°C until analysis. Plasma samples were processed by protein precipitation extraction, and the extracts were analyzed by LC/MS/MS. The results demonstrated that the compounds in Examples according to the present disclosure exhibit unexpectedly high blood-brain barrier penetration.

As shown in Table 4, at 0.5 hours post oral dosing, the brain-blood concentration ratio of the compound in Example 2 reached 5.08, indicating remarkably high brain penetration. In contrast, Deucravacitinib and control compound A showed very low brain penetration.

Table 4: Blood-brain concentration ratio of the compound in Example 2 in rats 0.5 h after oral administration

| PK parameters | Example 2 |
|---|---|
| concentration in plamsa (ng/mL) | 515 |
| concentration in brain tissue (ng/mL) | 2615 |
| brain/blood concentration ratio | 508% |

Similar tests were performed on the compounds in other Examples, and they also had unexpectedly high brain/blood concentration ratio.

Table 5: Blood-brain concentration ratios of the compounds in other Examples after administration

| Example | administration route | brain/blood concentration ratio |
|---|---|---|
| 1 | IV | 315% |
| 5 | IV | 121% |
| 6 | IV | 32.2% |
| control compound A | IV | 8.79% |
| control compound B | IV | 22.8% |
| Deucravacitinib | PO | 3.20% |

The compounds of the present disclosure represent the first known class of highly selective TYK2 allosteric inhibitors with blood-brain barrier penetration, and also the structural type with the strongest brain penetrating ability among the reported molecular structures.

The properties of Deucravacitinib, control compound A, and control compound B used for comparison herein are as follows,

wherein Deucravacitinib has the following structural formula:

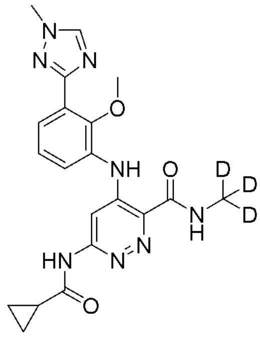

;

the control compound A has the following structural formula and can be prepared according to the method of Example 1 in document WO2022060973A1:

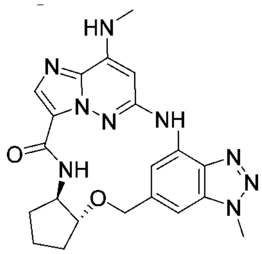

;

the control compound B has the following structural formula and can be prepared according to the method of Example 25 in document WO2020185755A1:

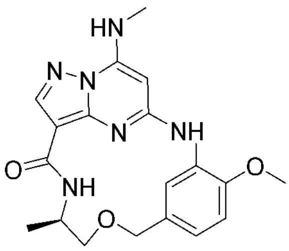

.

The above description combines preferred embodiments to explain the present disclosure, but these embodiments are only exemplary and serve only illustrative purposes. On this basis, various substitutions and modifications can be made to the present disclosure, all of which fall within the scope of protection of the present disclosure.

## Claims

**1.** A compound shown in Formula I, or pharmaceutically acceptable salts, hydrates, solvates, active metabolites, polymorphs, isotopic markers, isomers or prodrugs thereof:

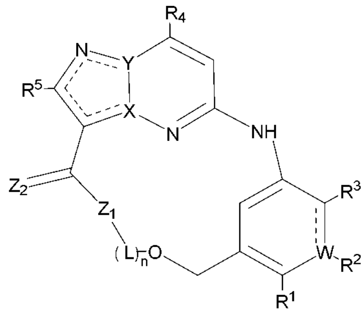

Formula I

wherein X and Y are each independently selected from the group consisting of N and C atoms, and one is N and the other is C;
W is selected from the group consisting of N and C atoms;
$Z_1$ is independently selected from the group consisting of O and S atoms;
$Z_2$ is selected from the group consisting of O, S and NR, wherein R is H or $C_{1-6}$ alkyl;
- - - - - - represents single or double bond;
$R^1$, $R^2$ and $R^3$ are each independently selected from the group consisting of hydrogen, halogen, oxo, $C_{1-6}$ alkyl, and $C_{1-6}$ alkoxy; or $R^2$ and $R^3$, together with the ring atoms to which they are attached, form a 5- or 6-membered heteroaryl ring substituted with 0-2 substituents $R^{23}$;
$R^4$ is selected from the group consisting of $NR^{41}R^{42}$, $C_{1-6}$ alkyl, and $C_{1-6}$ alkoxy;
$R^5$ is selected from the group consisting of hydrogen and $NR^{51}R^{52}$;
L is each independently selected from $-CR^{L1}R^{L2}-$;
$R^{L1}$ and $R^{L2}$ on each L are each independently selected from the group consisting of hydrogen and $C_{1-6}$ alkyl, or two $R^{L2}$ on the two adjacent L's, together with the carbons of L to which they are attached, form a $C_{3-6}$ cycloalkyl ring substituted with 0-2 $R^{23}$;
$R^{23}$ is each independently selected from the group consisting of halogen, $C_{1-6}$ alkyl, and $C_{1-6}$ alkoxy;
$R^{41}$ and $R^{42}$ are each independently selected from hydrogen and $C_{1-6}$ alkyl;
$R^{51}$ and $R^{52}$ are each independently selected from hydrogen and $C_{1-6}$ alkyl; and n is an integer from 2 to 5.

**2.** The compound or pharmaceutically acceptable salts, hydrates, solvates, active metabolites, polymorphs, isotopic markers, isomers or prodrugs thereof according to claim 1, wherein the compound has a structural formula of Formula IIa or Formula IIb:

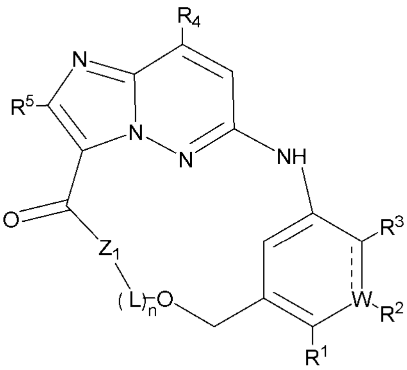

Formula IIa

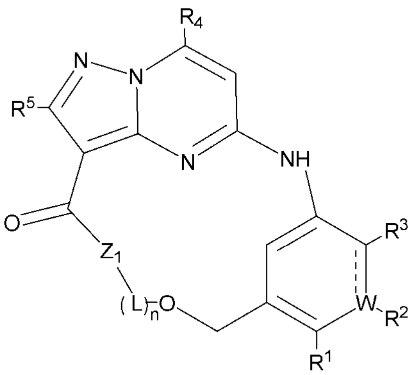

Formula IIb

wherein $R^1$, $R^2$, $R^3$, $R^4$, $R^5$, W, L, and n are as defined in claim 1.

**3.** The compound or pharmaceutically acceptable salts, hydrates, solvates, active metabolites, polymorphs, isotopic markers, isomers or prodrugs thereof according to claim 1 or 2, wherein $Z_1$ is O or S atom.

**4.** The compound or pharmaceutically acceptable salts, hydrates, solvates, active metabolites, polymorphs, isotopic markers, isomers or prodrugs thereof according to claim 1 or 2, wherein $R^4$ is $NR^{41}R^{42}$, $R^{41}$ is hydrogen and $R^{42}$ is $C_{1-3}$

alkyl group.

**5.** The compound or pharmaceutically acceptable salts, hydrates, solvates, active metabolites, polymorphs, isotopic markers, isomers or prodrugs thereof according to claim 1 or 2, wherein $R^4$ is $NR^{41}R^{42}$, $R^{41}$ is hydrogen and $R^{42}$ is methyl group.

**6.** The compound or pharmaceutically acceptable salts, hydrates, solvates, active metabolites, polymorphs, isotopic markers, isomers or prodrugs thereof according to claim 1 or 2, wherein $R^5$ is hydrogen or $-NH_2$.

**7.** The compound or pharmaceutically acceptable salts, hydrates, solvates, active metabolites, polymorphs, isotopic markers, isomers or prodrugs thereof according to claim 1 or 2, wherein n is 2 or 3; L is each independently selected from $-CR^{L1}R^{L2}-$, wherein $R^{L1}$ on each L is hydrogen, and $R^{L2}$ on each L is each independently selected from the group consisting of hydrogen and methyl.

**8.** The compound or pharmaceutically acceptable salts, hydrates, solvates, active metabolites, polymorphs, isotopic markers, isomers or prodrugs thereof according to claim 7, wherein among the (L)n formed by n L's, exactly one $R^{L2}$ is methyl.

**9.** The compound or pharmaceutically acceptable salts, hydrates, solvates, active metabolites, polymorphs, isotopic markers, isomers or prodrugs thereof according to claim 1 or 2, wherein n is 2 or 3; L is each independently selected from $-CR^{L1}R^{L2}-$, wherein $R^{L1}$ on each L is hydrogen, and two $R^{L2}$ on the two adjacent L's, together with the carbons of L to which they are attached, form a $C_{3-6}$ cycloalkyl ring.

**10.** The compound or pharmaceutically acceptable salts, hydrates, solvates, active metabolites, polymorphs, isotopic markers, isomers or prodrugs thereof according to claim 1 or 2, wherein (L)n formed by n L's is selected from $*-CH_2CH_2-**$, $*-CH_2CH_2CH_2-**$, $*-CH(CH_3)CH_2-**$, $*-CH_2CH(CH_3)-**$, $*-CH(CH_3)CH_2CH_2-**$,

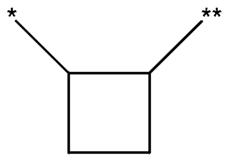

or

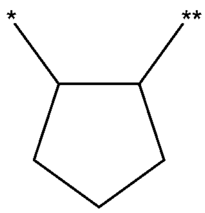

,

where * represents the site connected to atom $Z_1$, and ** represents the site connected to oxygen atom.

**11.** The compound or pharmaceutically acceptable salts, hydrates, solvates, active metabolites, polymorphs, isotopic markers, isomers or prodrugs thereof according to claim 1 or 2, wherein (L)n formed by n L's is $*-CH(CH_3)CH_2-**$; or

(L)n formed by n L's is

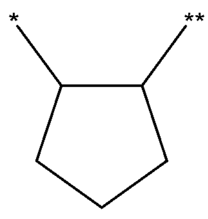

;

wherein * represents the site connected to atom $Z_1$, and ** represents the site connected to oxygen atom.

**12.** The compound or pharmaceutically acceptable salts, hydrates, solvates, active metabolites, polymorphs, isotopic markers, isomers or prodrugs thereof according to claim 1 or 2, wherein W is N, $R^1$ is H, $R^2$ is H or $C_{1-3}$ alkyl, and $R^3$ is oxo.

**13.** The compound or pharmaceutically acceptable salts, hydrates, solvates, active metabolites, polymorphs, isotopic markers, isomers or prodrugs thereof according to claim 1 or 2, wherein W is C, $R^1$ is H or halogen, $R^2$ is H or halogen, and $R^3$ is $C_{1-3}$ alkoxy.

**14.** The compound or pharmaceutically acceptable salts, hydrates, solvates, active metabolites, polymorphs, isotopic markers, isomers or prodrugs thereof according to claim 1 or 2, wherein W is C, the ------ between W and the carbon atom attached to $R^3$ is a double bond, $R^1$ is H, $R^2$ is F, and $R^3$ is methoxy.

**15.** The compound or pharmaceutically acceptable salts, hydrates, solvates, active metabolites, polymorphs, isotopic markers, isomers or prodrugs thereof according to claim 1 or 2, wherein W is C, the ------ between W and the carbon atom attached to $R^3$ is a double bond, $R^1$ is F, $R^2$ is H, and $R^3$ is methoxy.

**16.** The compound or pharmaceutically acceptable salts, hydrates, solvates, active metabolites, polymorphs, isotopic markers, isomers or prodrugs thereof according to claim 1 or 2, wherein W is C, the ------ between W and the carbon atom attached to $R^3$ is a double bond, $R^1$ is H, $R^2$ is H, and $R^3$ is methoxy.

**17.** The compound or pharmaceutically acceptable salts, hydrates, solvates, active metabolites, polymorphs, isotopic markers, isomers or prodrugs thereof according to claim 1 or 2, wherein the moiety

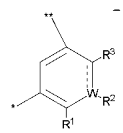

of Formula I, IIa, or IIb is

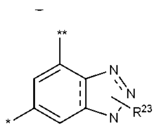,

wherein $R^{23}$ is selected from the group consisting of hydrogen and $C_{1-3}$ alkyl, * represents the site connected to $CH_2$, and ** represents the site connected to NH.

**18.** The compound or pharmaceutically acceptable salts, hydrates, solvates, active metabolites, polymorphs, isotopic markers, isomers or prodrugs thereof according to claim 17, wherein the moiety

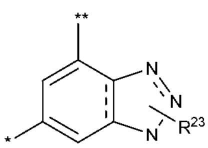

is

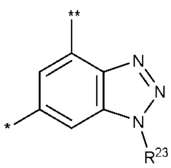, 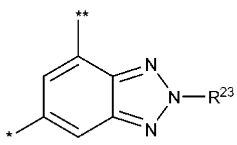, or 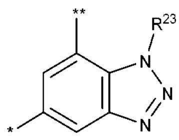,

wherein $R^{23}$ is selected from the group consisting of hydrogen and$C_{1-3}$ alkyl, * represents the site connected to $CH_2$, and ** represents the site connected to NH.

**19.** The compound or pharmaceutically acceptable salts, hydrates, solvates, active metabolites, polymorphs, isotopic markers, isomers or prodrugs thereof according to claim 17, wherein the moiety

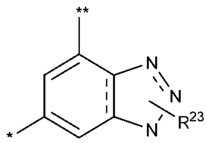

is

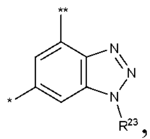,

wherein $R^{23}$ is methyl, * represents the site connected to $CH_2$, and ** represents the site connected to to NH.

**20.** The compound or pharmaceutically acceptable salts, hydrates, solvates, active metabolites, polymorphs, isotopic markers, isomers or prodrugs thereof according to claim 1 or 2, wherein the compound is selected from the following compounds:

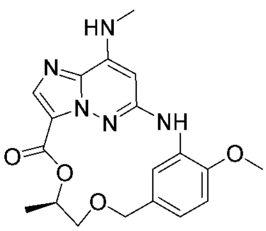
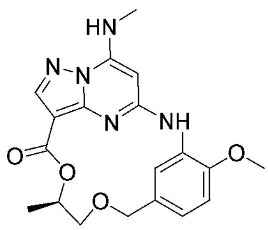
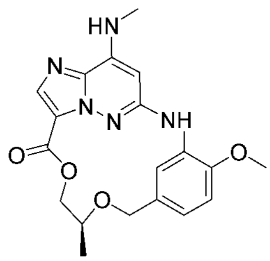

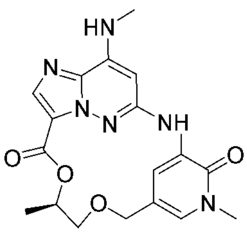
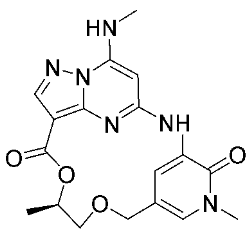
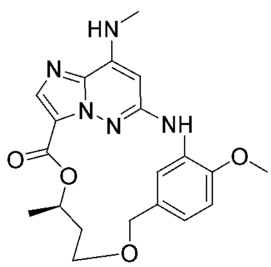

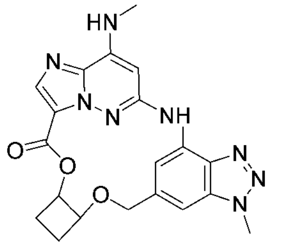
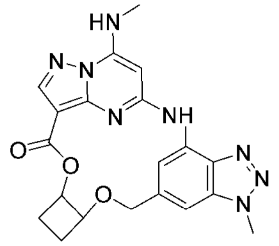
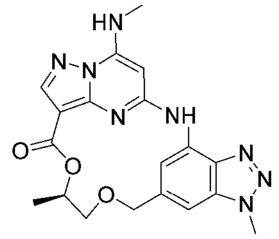
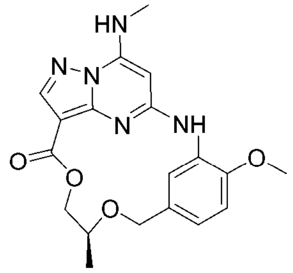
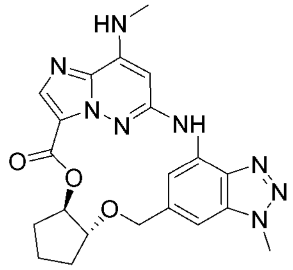
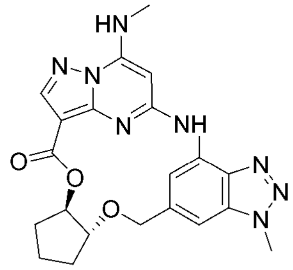
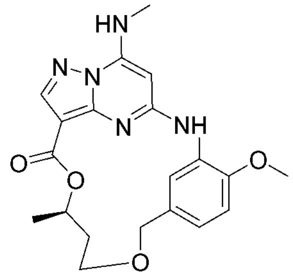
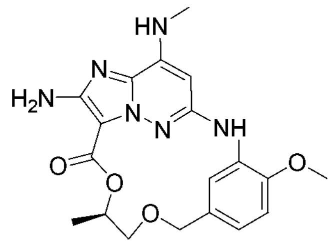
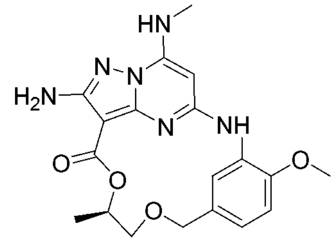
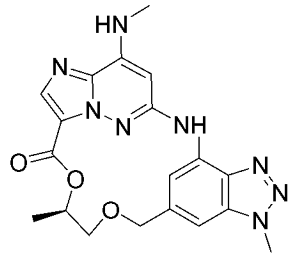
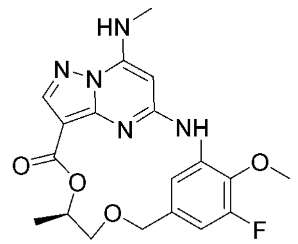
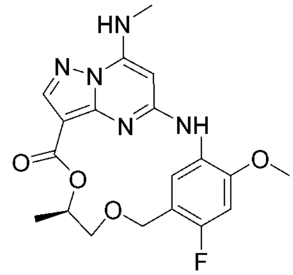

.

**21.** A pharmaceutical composition, comprising the compound or pharmaceutically acceptable salts, hydrates, solvates, active metabolites, polymorphs, isotopic markers, isomers or prodrugs thereof according to any one of claims 1 to 20, and a pharmaceutically acceptable carrier.

**22.** Use of the compound or pharmaceutically acceptable salts, hydrates, solvates, active metabolites, polymorphs, isotopic markers, isomers or prodrugs thereof according to any one of claims 1 to 20, and the pharmaceutical composition according to claim 18 in manufacture of pharmaceuticals for treating tyrosine kinase-mediated diseases.

**23.** Use according to claim 22, wherein the tyrosine kinase is selected from TYK2 kinases.

**24.** Use according to claim 22, wherein the tyrosine kinase-mediated diseases include inflammatory autoimmune diseases, tumors, and neurodegenerative diseases.

**25.** Use according to claim 22, wherein inflammatory autoimmune diseases are selected from the group consisting of atopic dermatitis, hidradenitis suppurativa, psoriasis, psoriatic arthritis, Crohn's disease, ulcerative colitis, lupus erythematosus, scleroderma, and autoimmune encephalopathy; tumors are selected from the group consisting of leukemia, lymphoma, myeloma, and brain tumor; and neurodegenerative diseases are selected from the group consisting of brain atrophy, dementia, Parkinson's syndrome, Alzheimer's disease, amyotrophic lateral sclerosis, and multiple sclerosis.

**26.** A method for treating tyrosine kinase-mediated diseases, comprising administering a therapeutically effective amount of the compound or pharmaceutically acceptable salts, hydrates, solvates, active metabolites, polymorphs, isotopic markers, isomers or prodrugs thereof according to any one of claims 1 to 20, or the pharmaceutical composition according to claim 18 to a patient in need of administration.

**INTERNATIONAL SEARCH REPORT**

International application No.
**PCT/CN2024/084966**

**A. CLASSIFICATION OF SUBJECT MATTER**

C07D498/22(2006.01)i; C07D498/18(2006.01)i; C07D487/22(2006.01)i; C07D487/18(2006.01)i; A61K31/5025(2006.01)i; A61P37/02(2006.01)i; A61P29/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT, CNKI, ENTXT, ISI, CAPLUS(STN), REGISTRY(STN): 北京普祺医药, 胡伟, 祝力, 戴丽光, 朱冰, 杜云龙, 酪氨酸激酶, 免疫, 肿瘤, 神经退行, 结构式检索, structural formula search, tyrosine kinase, TYK2, immun+, cancer, tumor, neurodeg+

**C. DOCUMENTS CONSIDERED TO BE RELEVANT**

| Category* | Citation of document, with indication, where appropriate, of the relevant passages | Relevant to claim No. |
|---|---|---|
| PX | WO 2023178235 A1 (ALUMIS INC.) 21 September 2023 (2023-09-21) description, paragraphs 15-122, and embodiments 1-47 | 1-26 |
| PX | WO 2023178234 A1 (ALUMIS INC.) 21 September 2023 (2023-09-21) description, paragraphs 15-151, and embodiments 1-5 | 1-26 |
| PX | WO 2023208244 A1 (MEDSHINE DISCOVERY INC.) 02 November 2023 (2023-11-02) description, page 1, paragraph 6 to page 34, paragraph 7, and embodiments 1-7 | 1-26 |
| X | CN 113811534 A (ESKER THERAPEUTICS, INC.) 17 December 2021 (2021-12-17) description, paragraphs 18-412, and embodiments 1-62 | 1-26 |
| X | WO 2022060973 A1 (ALUMIS, INC.) 24 March 2022 (2022-03-24) description, paragraphs 16-144, and embodiment 1 | 1-26 |
| X | ZHANG, Kuojun et al. "Development and Therapeutic Implications of Tyrosine Kinase 2 Inhibitors" *Journal of Medicinal Chemistry*, Vol. 66, No. (7), 23 March 2023 (2023-03-23), pp. 4378-4416 | 1-26 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 June 2024** | **20 June 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/084966

C. DOCUMENTS CONSIDERED TO BE RELEVANT

| Category* | Citation of document, with indication, where appropriate, of the relevant passages | Relevant to claim No. |
|---|---|---|
| X | CN 113874021 A (VENTYX BIOSCIENCES INC.) 31 December 2021 (2021-12-31) description, paragraphs 3-236, and embodiments 1-3 | 1-26 |
| A | WO 2019206069 A1 (BEIJING PUQI MEDICINE TECHNOLOGY CO., LTD.) 31 October 2019 (2019-10-31) description, page 2, line 14 to page 15, line 9, and embodiments 1-25 | 1-26 |
| A | WO 2022171139 A1 (BEIJING GUOHONG BIOMEDICAL TECHNOLOGY CO., LTD.) 18 August 2022 (2022-08-18) description, page 3, line 1 to page 21, line 6, and embodiments 1-41 | 1-26 |

Form PCT/ISA/210 (second sheet) (July 2022)

INTERNATIONAL SEARCH REPORT

International application No.
**PCT/CN2024/084966**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **22-26**
because they relate to subject matter not required to be searched by this Authority, namely:
Claims 22-26 relate to a method for treatment or prevention of a disease in a human or animal body. However, the present search report is provided regarding a pharmaceutical use of a compound or composition.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

## INTERNATIONAL SEARCH REPORT
**Information on patent family members**

International application No.
**PCT/CN2024/084966**

| Patent document cited in search report | Publication date (day/month/year) | Patent family member(s) | Publication date (day/month/year) |
|---|---|---|---|
| WO 2023178235 A1 | 21 September 2023 | None | |
| WO 2023178234 A1 | 21 September 2023 | None | |
| WO 2023208244 A1 | 02 November 2023 | None | |
| CN 113811534 A | 17 December 2021 | US 2022177486 A1 | 09 June 2022 |
| | | CA 3132632 A1 | 17 September 2020 |
| | | AU 2020239026 A1 | 23 September 2021 |
| | | KR 20210141973 A | 23 November 2021 |
| | | EP 3938369 A1 | 19 January 2022 |
| | | EP 3938369 A4 | 25 January 2023 |
| | | WO 2020185755 A1 | 17 September 2020 |
| | | IL 286248 A | 31 October 2021 |
| | | BR 112021017996 A2 | 16 November 2021 |
| | | JP 2022524974 A | 11 May 2022 |
| WO 2022060973 A1 | 24 March 2022 | CN 117279922 A | 22 December 2023 |
| | | CA 3192621 A1 | 24 March 2022 |
| | | US 2023357273 A1 | 09 November 2023 |
| | | JP 2023541203 A | 28 September 2023 |
| | | EP 4214215 A1 | 26 July 2023 |
| | | KR 20230069984 A | 19 May 2023 |
| | | AU 2021345181 A1 | 04 May 2023 |
| | | BR 112023004910 A2 | 06 June 2023 |
| | | IN 202317026763 A | 24 November 2023 |
| CN 113874021 A | 31 December 2021 | CA 3134814 A1 | 01 October 2020 |
| | | IL 286635 A | 31 October 2021 |
| | | MX 2021011723 A | 22 October 2021 |
| | | BR 112021019070 A2 | 15 February 2022 |
| | | WO 2020198379 A1 | 01 October 2020 |
| | | AU 2020247990 A1 | 11 November 2021 |
| | | US 2023406858 A1 | 21 December 2023 |
| | | US 2022169651 A1 | 02 June 2022 |
| | | US 11780842 B2 | 10 October 2023 |
| | | EA 202192625 A1 | 21 March 2022 |
| | | JP 2022529311 A | 21 June 2022 |
| | | SG 11202110523 XA | 28 October 2021 |
| | | KR 20220004641 A | 11 January 2022 |
| | | EP 3946350 A1 | 09 February 2022 |
| | | EP 3946350 A4 | 07 September 2022 |
| WO 2019206069 A1 | 31 October 2019 | EP 3786167 A1 | 03 March 2021 |
| | | EP 3786167 A4 | 09 June 2021 |
| | | US 2022162218 A1 | 26 May 2022 |
| | | JP 2021519828 A | 12 August 2021 |
| | | JP 7128345 B2 | 30 August 2022 |
| | | JP 2022126780 A | 30 August 2022 |
| WO 2022171139 A1 | 18 August 2022 | CN 116829562 A | 29 September 2023 |
| | | US 20240132517 A1 | 25 April 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022060973 A1
- WO 2020185755 A1


### Non-patent literature cited in the description


- **CAREY** ; **SUNDBERG**. Advanced Organic Chemistry. Plenum Press, 2000, vol. A
- **CAREY, SUNDBERG**. Advanced Organic Chemistry. Plenum Press, 2001, vol. B
- **M. CAIRA et al.** *J. Pharmaceutical Sci.*, 2004, vol. 93 (3), 601-611
- **E.C. VAN TONDER et al.** *AAPS PharmSciTech.*, 2004, vol. 5 (1)
- **AL BINGHAM et al.** *Chem. Commun.*, 2001, 603-604
- **T. HIGUCHI** ; **V. STELLA**. Pro-drugs as Novel Delivery Systems. *ACS Symposium Series*, 1987, vol. 14
- Bioreversible Carriers in Drug Design. American Pharmaceutical Association and Pergamon Press, 1987
- Design of Prodrugs. Elseview, 1985
- Method in Enzymology. Academic, 1985, vol. 42, 309-396
- Design and Application of Prodrugs. **BUNDGAARD, H.** A Textbook of Drug Design and Development. 1991, 113-191
- **BUNDGAARD, H.** *Advanced Drug Delivery Review*, 1992, vol. 8, 1-38
- **GOODMAN** ; **GILMAN**. The Pharmacological Basis of Therapeutics. Pergamon
- Remington's, Pharmaceutical Sciences. Mack Publishing Co.
- **GREENE** ; **WUTS**. Protective Groups In Organic Synthesis. Wiley and Sons, 1991